(19) Europäisches Patentamt
European Patent Office
Office européen des brevets

(11) EP 0 783 531 B1

(12) **FASCICULE DE BREVET EUROPEEN**

(45) Date de publication et mention de la délivrance du brevet:
**25.11.1998 Bulletin 1998/48**

(21) Numéro de dépôt: **95932805.5**

(22) Date de dépôt: **28.09.1995**

(51) Int Cl.6: **C08F 2/38**, B01F 17/00

(86) Numéro de dépôt international:
**PCT/FR95/01253**

(87) Numéro de publication internationale:
**WO 96/10045 (04.04.1996 Gazette 1996/15)**

(54) **POLYMERES FONCTIONNALISES PAR DES ACIDES AMINES OU DES DERIVES D'ACIDES AMINES, LEUR PROCEDE DE SYNTHESE, LEUR UTILISATION COMME AGENTS TENSIOACTIFS EN PARTICULIER DANS DES COMPOSITIONS COSMETIQUES ET NOTAMMENT DES VERNIS A ONGLES**

DURCH AMINOSÄUREN ODER DEREN DERIVATE FUNKTIONALISIERTE POLYMERE, VERFAHREN ZU IHRER HERSTELLUNG, IHRE VERWENDUNG ALS TENSIDE, INSBESONDERE IN KOSMETIKA UND ALS NAGELLACK

POLYMERS FUNCTIONALISED WITH AMINO ACIDS OR AMINO ACID DERIVATIVES, METHOD FOR SYNTHESISING SAME, AND USE THEREOF AS SURFACTANTS IN COSMETIC COMPOSITIONS, PARTICULARLY NAIL VARNISHES

(84) Etats contractants désignés:
**BE CH DE ES FR GB IT LI**

(30) Priorité: **28.09.1994 FR 9411576**

(43) Date de publication de la demande:
**16.07.1997 Bulletin 1997/29**

(73) Titulaire: **LVMH RECHERCHE**
**92752 Nanterre (FR)**

(72) Inventeurs:
- **TONDEUR, Carole**
  **F-68100 Mulhouse (FR)**
- **RIESS, Henri-Gérard**
  **F-68200 Mulhouse (FR)**
- **MEYBECK, Alain**
  **F-92400 Courbevoie (FR)**
- **TRANCHANT, Jean-François**
  **F-45760 Boigny-sur-Bionne (FR)**

(74) Mandataire: **Giraud, Françoise et al**
**Cabinet Beau de Loménie**
**158, rue de l'Université**
**75340 Paris Cedex 07 (FR)**

(56) Documents cités:
**EP-A- 0 160 103**
**EP-A- 0 207 026**
**DE-A- 1 545 883**
**US-A- 4 107 096**
**US-A- 5 298 585**

- **ANGEWANDTE MAKROMOLEKULARE CHEMIE, vol. 213, no. 3693, 1993 BASEL, page 55-63 XP 000416400 C.ERBIL ET AL 'free radical polymerisation of acrylamide...' cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 783 531 B1

## Description

La présente invention concerne de nouveaux polymères fonctionnalisés, leur procédé de synthèse et leurs utilisations comme agents tensioactifs, notamment comme agents mouillants et/ou dispersants et/ou stabilisants de dispersions de particules solides et pour préparer des microdispersions.

On connaît déjà des polymères fonctionnalisés en extrémité de chaîne et plus particulièrement des polymères fonctionnalisés obtenus par polymérisation en présence d'un agent transféreur de chaîne.

On citera plus particulièrement dans ce domaine les travaux de Y. YAMASHITA, Y. CHUJO et coll. qui ont essentiellement décrit la synthèse de polyméthacrylates de méthyle (PMMA) terminés par des groupements -OH ou -COOH obtenus en utilisant l'acide thiomalique comme agent de transfert de chaîne et leur utilisation dans la préparation de macromonomères et en tant que macromonomères pour polycondensations. Ces travaux sont relatés dans "Telechelic Polymers Synthesis and Applications" E.J. GOETHALS, CRC Press Inc. (1989) 169-179.

Y. YAMASHITA, Y. CHUJO, H. KOBAYASHI et KAWAKAMI décrivent dans Polym. Bull., 5, 361-366 (1981) des macromonomères de formule générale (PMMA) $SCH(COOH)CH_2COOH$ ainsi que d'autres macromonomères acryliques présentant la même fonction terminale. Tous ces macromonomères sont destinés à être utilisés dans des opérations de polycondensation.

Y. CHUJO, H. KOBAYASHI et Y. YAMASHITA décrivent dans J. of Polym. Sci., Part A : Polym. Chem., 27, 2007-2014 (1989) des macromonomères destinés également à des opérations de polycondensation, ces macromonomères étant constitués d'une chaîne polymérique PMMA terminée par un groupement fonctionnel aromatique dicarboxylique.

Dans tous ces documents, les macromonomères fonctionnalisés sont utilisés comme produits intermédiaires de réaction dans la préparation d'autres macromonomères ou directement comme macromonomères pour réaliser des réactions de polycondensation, ou de couplage.

Le brevet US 3 689 593 décrit des copolymères greffés dans lesquels les greffons sont constitués de macromonomères terminés par un groupement OH, COOH ou $NH_2$ sur lesquels ont réagi un diisocyanate, puis un monomère vinylique fonctionnel. Ces polymères sont utiles comme agents filmogènes dans des compositions de type peinture.

D'autres macromonomères dans lesquels le groupement fonctionnel est relié au groupement polymérique par un groupement isocyanate ont été décrits en particulier pour leur application à la dispersion et à la stabilisation de pigments. On citera tout particulièrement les références suivantes :

- H.L. JAKUBAUSKAS dans J. Coat. Techn., 58, n° 736, 71-82 (1986),
- F.N. JONES dans U.S. Patent 4,070,388,
- T.A. ASHE dans U.S. Patent 4,032,698,
- D.R. THOMPSON dans US Patent 3,788,996, et
- R.A. BRAUN dans US Patent 3,684,771.

Les composés décrits dans les différents documents ci-dessus sont constitués de différents types de chaînes polymériques fonctionnalisées par différents groupements terminaux. Ces macromonomères sont tous obtenus en fonctionnalisant une chaîne polymérique par exemple par un groupement -OH sur lequel on fait ensuite réagir un triisocyanate. On obtient ainsi une chaîne polymérique terminée par deux groupements isocyanates sur lesquels on peut ensuite greffer de l'acide thiomalique. Les dispersions réalisées par l'intermédiaire de ces produits sont extrêmement résistantes à la floculation, ce qui donne aux peintures dans lesquelles elles sont incorporées un pouvoir couvrant, une brillance ainsi qu'une résistance aux pertes de brillance améliorés. De plus, le taux de pigments peut être augmenté en gardant le même degré de fluidité qu'avec les dispersants classiques. Cependant, ces produits contiennent des isocyanates, ce qui peut s'avérer gênant dans une formulation cosmétique s'il y a des fonctions isocyanates résiduelles.

Candan Erbil, A. Bahattin Soydan, A. Zehra Aroguz et A. Segai Saraç ont décrit dans die Angewandte Makromolekulare Chemie 213, (1993), 55-63, (Nr 3693) des polymères fonctionnalisés en extrémité de chaîne par un aminoacide. Ces polymères sont obtenus par polymérisation radicalaire d'acrylamide en milieu acide amorcée par du sulfate de cérium en présence d'un α-aminoacide. Les travaux décrits dans cette publication résultent en fait de travaux plus anciens décrits dans les publications ci-dessous :

- G. MINO, S. KAIZERMAN et E. RASMUSSEN
  J. Polym. Sci., Vol. XXXVIII, 393-401(1959)

- S.V. SUBRAMANIAN et M. SANTAPPA
  J. Polym. Sci., Part A-1, 6, 493-504 (1968)

- A. SEZAI SARAC, CANDAN ERBIL et A. BAHATTIN SOYDAN

J. Appl. Polym. Sci., 44, 877-881 (1992)

Ainsi donc, le procédé décrit dans Candan Erbil, A. Bahattin Soydan, A. Zehra Aroguz et A. Sezai Saraç, Die Angew. Makromol. Chem., 213, 55-63 (1993) nécessite l'utilisation de solutions acides concentrées (acide sulfurique, nitrique,.... ou perchlorique) donc un milieu défavorable pour la plupart des dérivés d'acides aminés. Par ailleurs, Candan Erbil, A. Bahattin Soydan, A. Zehra Aroguz et A. Sezai Saraç ne décrivent que la synthèse de produits à chaîne polymérique hydrophile qui ne présentent donc pas de caractère tensioactif.

Par ailleurs, le brevet US-A-5 298 585 décrit des produits à caractère dispersant constitués de polymères fonctionnalisés en bout de chaîne par un sulfure d'amine et montre la supériorité de ces produits par rapport à des produits analogues fonctionnalisés, quant à eux, par un produit de type acide aminé. Toutefois, tous les produits décrits dans ce document, qui sont des produits nécessairement solubles dans l'eau, sont des produits constitués d'une chaîne polymérique essentiellement hydrophile et d'une tête polaire et ne doivent leur propriétés dispersantes qu'à la polarité de cette tête.

Par conséquent, les produits décrits dans ce document, tout comme ceux décrits dans l'article cité précédemment de Candan Erbil et al. ne présentent aucun caractère tensioactif.

La demanderesse a maintenant trouvé de nouveaux polymères fonctionnalisés par des acides aminés ou des dérivés d'acides aminés présentant une chaîne polymérique hydrophobe.

Ces produits présentent l'avantage d'avoir de remarquables propriétés tensioactives ce qui permet leur utilisation en particulier comme agents mouillants de surfaces ou de particules solides, et/ou dispersants et/ou stabilisants de dispersions de particules solides dans un milieu organique ainsi que pour la préparation de microdispersions de polymères en particulier pour la préparation de microgels ou de microlatex.

Ainsi, selon une de ses caractéristiques essentielles, l'invention concerne un polymère fonctionnalisé en bout de chaîne répondant à la formule :

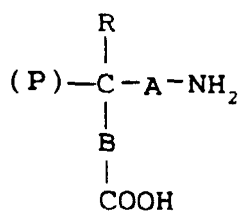

(1)

dans laquelle :

- la chaîne polymérique (P) est une chaîne hydrophobe obtenue par polymérisation radicalaire d'au moins un monomère,
- R représente un atome d'hydrogène ou une chaîne hydrocarbonée comprenant 1 à 8 atomes de carbone, linéaire ou ramifiée, éventuellement substituée par au moins un groupe choisi parmi $CO_2H$, $NH_2$, OH ou un groupe phényle, lui-même éventuellement substitué,
- A et B, identiques ou différents, représentent chacun une liaison simple, une chaîne hydrocarbonée saturée ou insaturée, linéaire ou ramifiée comprenant de 1 à 16 atomes de carbone, pouvant contenir une liaison amide, une chaîne peptidique comprenant 2 à 4 acides aminés, en particulier, naturels,
- les groupements $NH_2$ et/ou COOH étant libres ou salifiés.

Dans les polymères fonctionnalisés définis ci-dessus, la chaîne polymérique (P) a avantageusement une masse molaire moyenne en nombre comprise entre 500 et 250 000.

D'une façon générale, les produits de formule (1) de l'invention peuvent être obtenus par polymérisation radicalaire d'au moins un monomère conduisant à la formation de la chaîne polymérique hydrophobe (P) en présence d'un acide aminé ou d'un dérivé d'acide aminé répondant à la formule (2) ci-dessous :

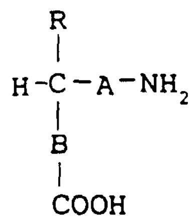

(2)

agissant comme agent de transfert de chaîne lors de ladite polymérisation radicalaire.

Dans la formule (2) ci-dessus, les groupements R, A et B ont les significations données pour les mêmes groupements dans la formule (1).

Le rôle d'agent de transfert de chaîne du produit de formule (2) dans la polymérisation radicalaire est rendu possible du fait du caractère labile de l'hydrogène porté par le carbone dans la formule (2) ci-dessus.

Ce procédé qui met en oeuvre un acide aminé ou un dérivé d'acide aminé comme agent de transfert de chaîne lors de la polymérisation radicalaire d'au moins un monomère conduisant à la formation d'une chaîne polymérique (P) est inspiré par analogie du procédé décrit dans les travaux de YAMASHITA, CHUJO et coll., cités précédemment, en remplaçant le thiol par un acide aminé ou un dérivé d'acide aminé présentant un atome d'hydrogène labile.

Plus précisément, comme cela a été signalé précédemment, la synthèse de PMMA fonctionnalisés par des groupements acides carboxyliques est déjà connue. YAMASHITA, CHUJO et coll. ont développé ce type de macromonomères dans le but de les copolymériser par polycondensation et ainsi former des copolymères greffés (voir en particulier, E.J. GOETHALS, "Telechelic Polymers Synthesis and Applications" CRC Press, Inc, 169-179 (1989)).

Le principe de cette synthèse consiste à polymériser du méthacrylate de méthyle par voie radicalaire en présence d'un agent de transfert portant les fonctions acides, en l'occurrence l'acide thiomalique, dans les conditions indiquées dans le schéma réactionnel (I) ci-dessous:

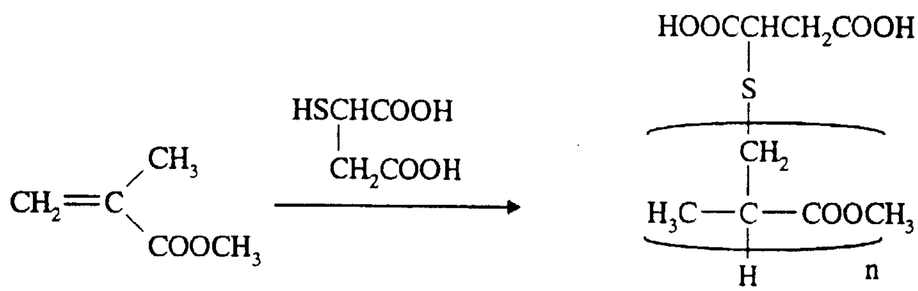

(I)

Cette réaction s'effectue en milieu solvant, par exemple THF, en présence d'un amorceur de polymérisation radicalaire, par exemple l'azobisisobutyronitrile (AIBN) à une température de l'ordre de 60°C.

Les polymères fonctionnalisés selon l'invention pourront avantageusement être préparés dans un procédé par analogie inspiré du schéma réactionnel (I) ci dessus, en choisissant le(s) monomère(s) et l'acide aminé ou le dérivé d'acide aminé en fonction du produit final visé.

La réaction de polymérisation radicalaire du (ou des) monomère(s) sera réalisée en milieu solvant, en présence d'un agent de polymérisation radicalaire constitué d'un amorceur organo-soluble, de préférence choisi dans la famille des amorceurs azoïques.

A titre d'exemple d'amorceur préféré, on citera l'azobisisobutyronitrile (AIBN).

La réaction a lieu en milieu solvant.

Le solvant ou mélange de solvants sera choisi en fonction de la nature du (ou des) monomère(s) à polymériser et de l'acide aminé ou du dérivé d'acide aminé utilisé.

Le solvant ou mélange de solvants sera choisi en fonction de la nature des réactifs De préférence il s'agira d'un solvant ou d'un mélange de solvants capable de dissoudre l'ensemble des réactifs en présence, à savoir les monomères, le polymère formé, l'amorceur et l'agent de transfert.

Le solvant peut avoir un caractère acide, on utilisera, par exemple, l'acide acétique; il peut également avoir un caractère basique, par exemple, la diméthyléthanolamine

La température de réaction sera avantageusement comprise entre 30°C et 120°C, mais est à ajuster en fonction des réactifs en présence. On comprend aisément qu'elle dépend de la nature de l'amorceur et de la nature du solvant.

La masse moléculaire du polymère fonctionnalisé résultant du procédé décrit ci-dessus sera contrôlée en jouant sur la quantité d'agent transféreur de chaîne introduite.

Les proportions d'amorceur, transféreur et monomère(s) peuvent être calculées d'après la relation classique connue pour le transfert de chaîne :

$$\frac{1}{DPn} = \frac{1}{DPn_0} + Cs(\frac{S}{M})$$

où

- S/M est le rapport molaire agent de transfert de chaîne/monomère à engager
- Cs est la constante de transfert dépendant de la nature du (des) monomère(s), de l'agent de transfert, de la température et du solvant
- DPn est le degré de polymérisation du polymère que l'on veut synthétiser
- $DPn_0$ est le degré de polymérisation du polymère que l'on aurait obtenu en absence d'agent de transfert.

Ainsi donc, selon un autre de ses aspects, l'invention concerne un procédé de préparation des polymères fonctionnalisés selon l'invention consistant à réaliser la polymérisation radicalaire d'un monomère conduisant à une chaîne polymérique (P) hydrophobe en présence d'un acide aminé ou d'un dérivé d'acide aminé agissant comme agent de transfert de chaîne lors de la polymérisation radicalaire et répondant à la formule (2)

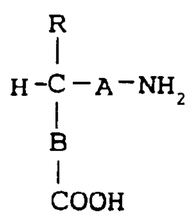

(2)

dans laquelle :

- R représente un atome d'hydrogène ou une chaîne hydrocarbonée comprenant 1 à 8 atomes de carbone, linéaire ou ramifiée, éventuellement substituée par au moins un groupe choisi parmi $CO_2H$, $NH_2$, OH ou un groupe phényle, lui-même éventuellement substitué,
- A et B, identiques ou différents, représentent chacun une liaison simple, une chaîne hydrocarbonée saturée ou insaturée, linéaire ou ramifiée comprenant de 1 à 16 atomes de carbone, pouvant contenir une liaison amide, une chaîne peptidique comprenant 2 à 4 acides aminés, en particulier, naturels.

Ainsi donc, à titre d'exemples de produits intéressants selon l'invention, on citera les produits répondant aux formules suivantes :

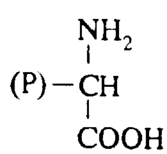

(3)

obtenu en utilisant la glycine $H_2N-CH_2-COOH$ comme agent de transfert de chaîne,

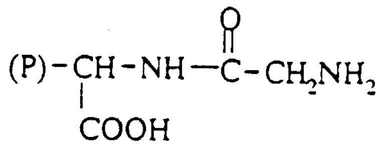 (4)

et

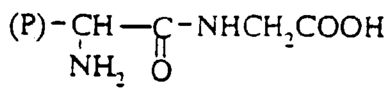 (5)

obtenus en utilisant la glycylglycine,

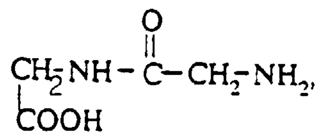

comme agent de transfert de chaîne, ce produit présentant deux carbones portant des hydrogènes relativement labiles.

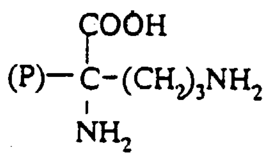 (6)

obtenu en utilisant l'ornithine

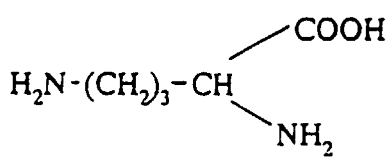

comme agent de transfert de chaîne.

Une sous-famille de produits selon l'invention correspond aux produits répondant à la formule (7) ci-dessous :

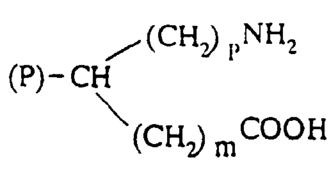 (7)

dans laquelle

- P désigne une chaîne polymérique hydrophobe obtenue par polymérisation radicalaire d'au moins un monomère,
- m et p sont des entiers compris entre 0 et 11 et dont la somme est comprise entre 2 et 11.

Ces produits de formule (7) sont avantageusement préparés par un procédé comprenant :

- une étape de polymérisation radicalaire d'au moins un monomère conduisant à une chaîne polymérique hydrophobe (P) en présence d'un lactame agissant comme agent de transfert de chaîne et répondant à la formule (8) :

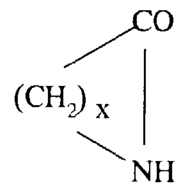

(8)

dans laquelle x est un entier compris entre 3 et 12,
- suivie d'une deuxième étape d'hydrolyse du produit obtenu lors de la première étape.

Le produit intermédiaire résultant de la première étape du procédé ci-dessus est lui-même brevetable en soi en tant que produit intermédiaire nouveau.

Un tel produit répond à la formule (9) :

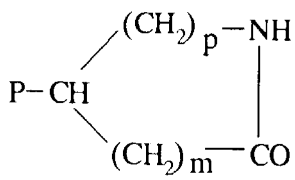

(9)

dans laquelle :

- P est une chaîne polymérique hydrophobe résultant de la polymérisation radicalaire d'un monomère acrylique ou vinylique,
- p et m sont des entiers compris entre 0 et 11 et dont la somme est comprise entre 2 et 11.

Dans tous les polymères décrits précédemment, la chaîne polymérique hydrophobe (P) pourra être constituée de toute chaîne polymérique susceptible d'être obtenue par polymérisation radicalaire d'au moins un monomère.

Il pourra donc s'agir aussi bien d'une chaîne constituée par polymérisation radicalaire d'un seul type de monomère que d'un mélange de plusieurs monomères différents.

On choisira avantageusement comme monomère, un monomère acrylique ou vinylique.

Parmi les monomères acryliques, on citera tout particulièrement les acrylates, méthacrylates, éthylacrylates d'un groupe hydrocarboné en C1 à C18, saturé ou insaturé, en particulier allylique, linéaire, ramifié ou comprenant un cycle.

Un monomère préféré selon l'invention est le méthacrylate de méthyle. La chaîne polymérique sera alors constituée de polyméthacrylate de méthyle (PMMA).

Parmi les monomères vinyliques, on citera notamment le styrène, l'alpha-méthyl styrène, les styrènes substitués, l'acrylonitrile, les esters vinyliques tels que l'acétate de vinyle.

Parmi les mélanges de monomères, on citera en particulier les mélanges d'acrylate ou de méthacrylate d'alkyle et d'acrylate ou de méthacrylate d'allyle, plus particulièrement les mélanges de méthacrylate de méthyle et de méthacrylate d'allyle. L'avantage de tels mélanges de monomères est qu'ils conduisent à des chaînes polymériques partiellement insaturées permettant d'obtenir des propriétés spécifiques des polymères liées à la présence de ces insaturations dans la chaîne polymérique.

Comme on l'a vu précédemment, on peut jouer sur la longueur de la chaîne polymérique en jouant notamment sur la proportion d'acide aminé ou de dérivé d'acide aminé agissant comme agent de transfert de chaîne.

Toutefois, les composés les plus intéressants selon l'invention sont ceux pour lesquels la chaîne polymérique (P) a une masse molaire moyenne en nombre comprise entre 500 et 250.000.

Tous les polymères fonctionnalisés précédents présentent d'intéressantes propriétés tensioactives.

Plus précisément, tous les polymères fonctionnalisés précédemment décrits permettent, du fait de leurs propriétés

tensioactives, d'abaisser la tension superficielle ou interfaciale, qu'il s'agisse d'un système liquide/liquide ou liquide/ solide.

En particulier, ces produits lorsqu'ils sont mis en contact avec une surface permettent d'abaisser son énergie de surface.

Ces produits lorsqu'ils sont mis en présence d'une charge de particules permettent également d'abaisser l'énergie de surface desdites particules. Ce phénomène d'abaissement de l'énergie de surface des particules se traduit par un effet mouillant du polymère par rapport à ladite particule, par un effet dispersant desdites particules lorsque ces particules se trouvent en suspension dans un solvant et par un effet stabilisant des dispersions de particules dans un solvant.

Pour ces différents types d'effets tensioactifs dans des systèmes liquide/solide, les polymères fonctionnalisés peuvent être sous forme salifiée ou non.

Les propriétés tensioactives des polymères fonctionnalisés décrits ci-dessus peuvent également se manifester dans des systèmes liquide/liquide.

A titre d'exemple de tels systèmes liquide/liquide on citera des émulsions huile-dans-l'eau, des émulsions eau-dans-l'huile ainsi que des systèmes constituant les systèmes de départ destinés à être polymérisés pour préparer un latex.

Dans le cas de ces systèmes liquide/liquide, le polymère fonctionnalisé peut être soit sous forme salifiée, soit sous forme libre, mais il sera de préférence sous forme salifiée, ce qui augmente son pouvoir émulsifiant.

Ainsi donc, tous les polymères fonctionnalisés précédents présentent d'intéressantes propriétés tensioactives permettant en particulier leur utilisation en tant qu'agents mouillants, et/ou dispersants et/ou stabilisants de dispersions de particules solides, permettant ainsi la formation de dispersions stables de particules.

Par ailleurs, ces mêmes polymères fonctionnalisés après neutralisation, le cas échéant, des groupements COOH ou $NH_2$ présentent d'intéressantes propriétés tensioactives, permettant en particulier leur utilisation pour la préparation de microdispersions utilisables en particulier pour la préparation de microgels ou de microlatex.

Ainsi donc selon un autre de ses aspects, l'invention concerne l'utilisation des polymères fonctionnalisés répondant à la formule (1) telle que définie précédemment et dans lesquels la chaîne polymérique hydrophobe (P) a une masse molaire moyenne en nombre comprise entre 500 et 250.000, comme agents tensioactifs.

La demanderesse a plus particulièrement mis en évidence les propriétés tensioactives des polymères fonctionnalisés définis précédemment en étudiant leur effet sur l'énergie de surface d'un solide, en particulier d'une particule.

Elle a également mis en évidence leur caractère mouillant ainsi que leurs propriétés dispersantes et stabilisantes de dispersions de particules.

Les études de la demanderesse l'ont conduite, pour les différents polymères fonctionnalisés de la formule (1), en fonction de la nature du milieu dans lequel on souhaite disperser la poudre ainsi que de la nature de la poudre:

- d'une part, à déterminer le rendement d'adsorption du polymère fonctionnalisé défini comme la quantité de polymère fonctionnalisé "fixé" par rapport à la quantité engagée. Par polymère fonctionnalisé "fixé", on entend le polymère fonctionnalisé adsorbé par des liaisons physiques sur la surface de la particule solide,
- et d'autre part, à déterminer l'efficacité de l'agent tensioactif en tant qu'agent mouillant et/ou dispersant et/ou stabilisant.

Les études de la demanderesse l'ont amenée, en particulier, à mettre en évidence l'existence d'un palier dans les courbes , dites isothermes d'adsorption, représentant le taux d'adsorption du polymère fonctionnalisé en fonction de la concentration initiale du polymère fonctionnalisé à une température donnée.

L'efficacité de l'agent mouillant et/ou dispersant et/ou stabilisant est caractérisée par la quantité minimale de polymère fonctionnalisé à engager pour atteindre le palier de l'isotherme d'adsorption et recouvrir toute la surface de la particule.

Les études de la demanderesse l'ont conduite à mettre en évidence la réduction significative du paramètre de mouillabilité des particules solides. Cet effet met en évidence le caractère mouillant des produits selon l'invention.

Ainsi donc, l'invention concerne également l'utilisation des agents tensioactifs définis précédemment comme agent mouillant d'une surface solide ou d'une particule solide.

La demanderesse a également mis en évidence la réduction significative de la quantité d'agrégats formés entre les particules et/ou de leur taille en présence de polymère fonctionnalisé dans le milieu. Cet effet met en évidence le caractère dispersant des produits selon l'invention.

L'invention concerne donc également l'utilisation des polymères fonctionnalisés de la formule (1) dans lesquels la masse molaire moyenne en nombre de la chaîne polymérique est comprise entre 500 et 250.000 comme agents dispersants de particules solides dans un milieu organique.

La demanderesse a également mis en évidence que les polymères fonctionnalisés définis ci-dessus constituaient de remarquables agents stabilisants de dispersions de particules solides dans les milieux organiques et permettaient

en particulier d'obtenir des dispersions dont le surnageant ne devient pas limpide avant au moins 24 heures pour des teneurs en polymères correspondant au palier de l'isotherme d'adsorption.

Les particules solides peuvent être de tout type et ont avantageusement des dimensions comprises entre quelques nanomètres et quelques millimètres, de préférence entre 50 nm et 100 µm.

Il peut s'agir en particulier de particules minérales, en particulier de particules d'oxydes métalliques, par exemple de $TiO_2$, $SiO_2$, $Al_2O_3$, d'oxydes de fer, tels que goethite, hématite ou magnétite.

Il peut également s'agir de particules d'oxyde métallique recouvert de molécules de colorant organique.

Il peut également s'agir de particules organiques, par exemple de type rosinate, coprécipitées avec un colorant organique.

Le milieu organique utilisé pour réaliser le traitement des particules solides peut être tout solvant de la chaîne polymérique (P).

A titre d'exemple lorsque la chaîne polymérique est constituée de PMMA on choisira avantageusement le solvant parmi les esters tels que l'acétate de méthyle, d'éthyle, de butyle ou d'amyle, des cétones telles que l'acétone, la méthyl éthyl cétone ou la cyclohexanone, des solvants chlorés tels que le chloroforme ou le dichlorométhane, ou d'autres tels que le toluène, l'acide acétique.

On pourra également utiliser des mélanges d'un solvant du PMMA avec un non-solvant du PMMA dans des proportions gardant cette séquence soluble, comme par exemple un mélange acétate de butyle/éthanol ou isopropanol contenant au moins 50 % d'acétate de butyle.

Tous les polymères fonctionnalisés décrits précédemment ont un effet mouillant et dispersant.

L'effet stabilisant de la dispersion n'est, quant à lui, sensible qu'à partir d'une masse moléculaire moyenne en nombre du polymère supérieure à 1000, de préférence comprise entre 5 000 et 150 000.

Toutefois, on comprendra aisément que ces chaleurs dépendent à la fois de la nature de la charge à disperser ainsi que de celle de la tête polaire du polymère fonctionnalisé.

L'invention s'applique tout particulièrement à la dispersion des particules d'oxyde de titane, en particulier des particules de dimensions comprises entre 50 nm et 1 µm.

Dans la pratique, on détermine le rendement d'adsorption de la façon suivante : une masse $m_s$ de solution de concentration initiale Ci en polymère est mise en contact avec une masse $m_c$ de particules de surface spécifique S. Après adsorption, on détermine, après élimination des particules par centrifugation, la nouvelle concentration (Ce) de la solution. Le rendement (r) est donné par la formule :

$$r = (Ci\text{-}Ce) / Ci \times 100\ (\%)$$

Le taux d'adsorption (t) est donné, lui, par:

$$t = [(Ci\text{-}Ce) \times m_s] / m_c \times S$$

La dispersibilité est évaluée à partir des mesures de tailles des particules, par exemple à l'aide d'un appareil de type COULTER LS130.

Un avantage particulier des polymères fonctionnalisés utilisés comme dispersants selon l'invention est qu'ils conduisent à des dispersions stables.

Ainsi donc selon un autre de ses aspects, l'invention concerne des dispersions stables de particules solides dans un solvant ou un mélange de solvants, dans lesquelles l'agent dispersant est un polymère fonctionnalisé répondant à la formule (1) telle que définie précédemment et de masse molaire moyenne en nombre compris entre 500 et 250.000, ledit solvant ou mélange étant un solvant de ladite chaîne polymérique.

La stabilisation des dispersions est appréciée soit par mesure de la vitesse de sédimentation des suspensions en fonction du temps, soit par suivi des variations de l'absorbance du surnageant en fonction de la durée d'une centrifugation.

Un avantage des dispersions selon l'invention est que, lorsqu'il se produit une décantation au cours du temps, le dépôt formé reste facilement redispersable dans le milieu après agitation, le polymère recouvrant totalement la surface de la particule, empêchant ainsi son agrégation.

Un autre avantage des dispersions selon l'invention est que, lorsqu'elles sont séchées à température ambiante, la poudre récupérée peut être facilement redispersée par la suite dans le solvant.

Selon une autre de ses caractéristiques essentielles, l'invention concerne également toute composition renfermant les dispersions précédemment décrites. Elle concerne tout particulièrement les compositions contenant des dispersions de pigments destinées au domaine de la cosmétique.

Tous les produits décrits précédemment en tant qu'agent mouillants et/ou dispersants et/ou stabilisants de dispersion de particules, peuvent être utilisés également en tant qu'agents tensioactifs, ayant le rôle d'émulsifiants après neutralisation, le cas échéant, des groupements COOH ou $NH_2$.

L'invention concerne également l'utilisation des mêmes polymères pour la préparation de microdispersions de polymères :

- soit en milieu aqueux ou hydro-alcoolique (que nous désignerons par microlatex)
- soit en milieu organique (que nous désignerons par microgels) mettant en oeuvre ainsi leurs propriétés tensioactives.

Pour cette application, on choisira de préférence des polymères fonctionnalisés selon l'invention présentant une masse molaire moyenne en nombre ($M_n$) inférieure à environ 20 000, de préférence comprise entre 500 et 10 000.

Du fait de leurs propriétés tensioactives, les polymères fonctionnalisés utiles selon l'invention peuvent aisément former des solutions micellaires en milieu aqueux, après neutralisation de leurs fonctions acides et/ou basiques.

Ces solutions micellaires peuvent ensuite être utilisées dans la formulation de latex, en particulier de latex réticulés, notamment des latex réticulés de faibles tailles désignés par microlatex (10-150 nm). Ces microlatex peuvent être préparés par tout procédé de polymérisation en émulsion en présence d'un amorceur organosoluble ou hydrosoluble, comme l'a montré W. FUNKE avec des polyesters saturés ou insaturés, en particulier dans :

- "Emulsifying properties of saturated polyesters"
  H. BAUMANN, B. JOOS, W. FUNKE, Makromol. Chem., 187, 2933 (1986),

- "Saturated polyesters as emulsifiers for emulsion copolymerization of unsaturated polyester resins with styrene"
  H. BAUMANN, B. JOOS, W. FUNKE
  Makromol. Chem., 190, 83-92 (1989),

- "Reactor Microgels by Self-emulsifying Copolymerization of Unsaturated Polyester Resins with Acrylic and Methacrylic Esters"
  Makromol. Chem., 184, 755-762 (1983)
  M. MIYATA, W. FUNKE

- "Reactive Microgels by Emulsion Polymerization of Unsaturated Polyester Resins"
  Y.-Ch. YU, W. FUNKE
  Die Angewandte Makromol. Chem., 103, 187-202 (1982),

- "Surfactant Properties of Unsaturated Polyesters"
  Y.-Ch. Yu, W. FUNKE
  Die Angewandte Makromol. Chem., 103, 203-215 (1982).

L'avantage de ces microlatex est que :

- ils donnent des dispersions très stables de polymère,
- une fois séchés, on obtient des poudres de surfaces spécifiques extrêmement élevées;
- ils permettent de préparer des microgels ;
- ils permettent également de former des films (suivant la nature du polymère et du solvant).

Ces microlatex peuvent ensuite avantageusement être utilisés pour la préparation de microgels.

Ces microgels sont obtenus à partir des microlatex réticulés précédents, soit en transférant les particules constituant la dispersion dans un solvant après séchage préalable de la dispersion, soit par transfert azéotropique, soit par mélange avec un solvant puis distillation de l'eau.

A titre d'exemples de solvants, on citera les solvants aromatiques, les solvants chlorés tels que le chloroforme ou le chlorure de méthylène, des cétones et des esters tels que les acétates d'alkyle en $C_2$ à $C_4$, plus particulièrement l'acétate de butyle et l'acétate d'éthyle.

Les microgels de l'invention s'avèrent particulièrement utiles dans les domaines de la peinture et de la cosmétique, où ils permettent d'ajuster les caractéristiques rhéologiques. Il est en effet possible de mélanger les microgels à des formulations à base de nitrocellulose à extrait sec élevé, sans augmenter notablement la viscosité du système qui en résulte.

Le fait que les produits soient compatibles avec la nitrocellulose dissoute dans l'acétate de butyle en donnant un film transparent et brillant permet d'envisager leur utilisation pour faire des vernis, particulièrement des vernis à ongles.

Plus précisément, l'utilisation des microgels de l'invention dans des formulations de vernis et en particulier de vernis à ongles présente les avantages suivants :

- elle permet une amélioration des propriétés rhéologiques permettant, en particulier, d'éviter la précipitation des pigments, et une amélioration de la reproductibilité de ces propriétés. Ceci permet, en particulier, de diminuer sensiblement, voire même de supprimer, la quantité d'argiles organophiles généralement utilisées à cet effet mais dont les inconvénients sont bien connus,
- elle permet d'augmenter l'extrait sec du film constitué par le vernis, sans pour autant augmenter sensiblement la viscosité du vernis;
- elle confère plus de brillant au film,
- elle renforce l'effet thixotropique apporté par la bentone en milieu acétate.

La proportion pondérale de microgel selon l'invention, dans la composition finale du vernis, peut s'élever jusqu'à 30 % environ, par exemple dans le cas où on chercherait à diminuer, voire supprimer la quantité de nitrocellulose. Mais, en général, on préfère utiliser des proportions comprises entre 1 et 20 % en poids environ.

Comme cela ressort de l'exposé précédent, les dispersions de particules décrites ci-dessus ainsi que les microgels peuvent avantageusement être introduits dans différentes compositions, notamment des compositions cosmétiques et en particulier des vernis à ongles.

Ainsi donc, selon d'autres aspects, l'invention concerne également des compositions, notamment des compositions cosmétiques contenant des polymères fonctionnalisés selon l'invention.

En particulier, ces compositions cosmétiques sont destinées au soin ou au maquillage des ongles, et contiennent des dispersions de particules, en particulier de pigments, décrites ci-dessus.

L'invention concerne également des microlatex et microgels décrits précédemment ainsi que les compositions, notamment les compositions cosmétiques en contenant.

Elle concerne tout particulièrement des compositions contenant en outre de la nitrocellulose, en particulier les vernis à ongles.

Elle concerne également, d'une façon plus générale, l'utilisation des polymères de la famille (1) pour la préparation d'une composition, en particulier d'une composition de type peinture, ou d'une composition alimentaire, phytosanitaire ou cosmétique, en particulier d'une composition cosmétique destinée au soin ou au maquillage des ongles et plus particulièrement d'un vernis à ongles.

L'invention concerne plus particulièrement, comme cela ressort de l'exposé précédent, l'utilisation des polymères définis précédemmnent pour préparer des compositions contenant des particules solides en suspension, en particulier des pigments, dans lesquels ledit polymère fonctionnalisé est mis en présence desdites particules solides, notamment pigment, pour faciliter leur dispersion dans la composition.

Dans les compositions décrites ci-dessus, la quantité de polymères fonctionnalisés utilisés est comprise avantageusement entre 2 % et 7 % en poids par rapport au poids total des particules solides dispersées dans la composition.

Dans les compositions définies ci-dessus, le polymère fonctionnalisé est avantageusement compris dans des microdispersions de polymères, notamment des microgels ou des microlàtex, le polymère fonctionnalisé étant comme cela a été exposé précédemment utilisé comme agent tensioactif pour la préparation de ces microgels ou microlatex.

La quantité de microdispersions de polymères, de microgels ou de microlatex, est comprise avantageusement entre 1 % et 20 % en poids par rapport au poids total de la composition.

Selon un dernier aspect, l'invention concerne, comme cela ressort de l'exposé précédent, des compositions cosmétiques et, tout particulièrement, des compositions cosmétiques destinées au soin ou au maquillage des ongles, contenant des polymères fonctionnalisés définis précédemment, en particulier compris dans des microdispersions, notamment des microgels ou des microlatex.

Elle concerne tout particulièrement des compositions cosmétiques contenant en outre de la nitrocellulose.

## EXEMPLES

Les exemples ci-dessous sont donnés à titre purement illustratif de l'invention.

Ils sont donnés en référence aux figures 1 à 10 qui illustrent les propriétés dispersantes et stabilisantes des produits.

Les figures 1, 2a à 2d, 3 sont données en référence à l'exemple 1 et concernent plus précisément :

- figure 1 : l'isotherme et le rendement d'adsorption du polymère fonctionnalisé obtenu à l'exemple 1 sur une poudre de $TiO_2$ en milieu acétate de butyle,
- figures 2a, 2b, 2c, 2d : les résultats obtenus par mesure au COULTER LS130 de la dispersibilité de la même poudre dans le même milieu pour différentes concentrations initiales en polymère obtenu selon l'exemple 1,

- figure 3 : l'absorbance de la suspension en fonction du temps de centrifugation pour la poudre non traitée et en présence de différentes concentrations en polymère fonctionnalisé obtenu selon l'exemple 1,

Les figures 4, 5a à 5d, 6 et 7 sont donnés en référence à l'exemple 2 et concernent respectivement :

- figure 4 : l'isotherme et le rendement d'adsorption de l'un des polymères obtenus selon l'exemple 2 sur une poudre de $TiO_2$ en milieu acétate de butyle,
- figures 5a, 5b, 5c, 5d : les résultats obtenus par mesure au COULTER LS130 de la dispersibilité de la même poudre dans le même milieu pour différentes concentrations initiales du même polymère,
- figure 6 : l'absorbance de la suspension en fonction du temps de centrifugation pour la même poudre non traitée et en présence de différentes concentrations du même polymère fonctionnalisé,
- figure 7 : l'absorbance de la suspension en fonction du temps de centrifugation pour la même poudre non traitée en présence de différentes concentrations de l'autre polymère fonctionnalisé obtenu selon l'exemple 2,

Les figures 8, 9a, 9b, 9c et 10 sont données en référence à l'exemple 3 et concernent plus précisément :

- figure 8 : l'isotherme et le rendement d'adsorption du polymère non fonctionnalisé obtenu à l'exemple 3 sur une poudre de $TiO_2$ en milieu acétate de butyle,
- les figures 9a, 9b, 9c : les résultats obtenus par mesure au COULTER LS130 de la dispersibilité de la même poudre dans le même milieu pour différentes concentrations initiales en polymère obtenu selon l'exemple 3,
- figure 10 : l'absorbance de la suspension en fonction du temps de centrifugation pour la poudre non traitée et la même poudre en présence de différentes concentrations en polymère non fonctionnalisé obtenu selon l'exemple 3.

Exemple 1 polyméthacrylate de méthyle fonctionnalisé par l'ornithine 1.a. Synthèse

- 10 g d'omithine sont solubilisées dans 39 g d'eau,
- 136 g d'acide acétique sont ensuite versés progressivement.

La solution obtenue est placée dans un réacteur double enveloppe équipé d'une ancre d'agitation, d'un réfrigérant et d'une circulation d'azote.

- 0,16 g d'AIBN sont solubilisés dans 10 g de MMA.

Le tout est ajouté à la solution précédente.

L'ensemble est chauffé à 60°C durant 4 h. Après 2 h 30 de polymérisation, on observe une précipitation partielle du polymère.

Le produit précipité et celui resté en solution sont recueillis séparément.

Ils sont précipités dans le n-butanol. Séchés, éventuellement lavés à l'eau, ils sont ensuite solubilisés dans l'acétone et reprécipités dans l'eau.

Leur masse moléculaire en nombre est déterminée par GPC.

- Pour le polymère précipité lors de la polymérisation, Mn = 87.500,
- Pour le polymère resté en solution lors de la polymérisation, Mn = 53.200.

1.b. Etude de l'influence d'un PMMA fonctionnalisé par l'ornithine et de Mn = 87.500 sur la dispersion d'une poudre d'oxyde de titane en milieu acétate de butyle

La poudre utilisée est une poudre d'oxyde de titane de 200 nm de diamètre moyen et de 10 $m^2$/g de surface spécifique.

Le polymère fonctionnalisé de Mn = 87.500 préparé dans l'exemple 1.a est mis en solution dans l'acétate de butyle, à différentes concentrations. On ajoute ensuite 3g de $TiO_2$ à 10g de solution. On laisse alors agiter 24 h. La détermination des isothermes d'adsorption (taux d'adsorption en fonction de la concentration initiale du polymère à 20°C) permet d'obtenir le taux d'adsorption maximum ainsi que le rendement comme le montre la figure 1.

La figure 1 donne le taux d'adsorption (t) et le rendement d'adsorption (r) en fonction de la concentration initiale en polymère, exprimée en pourcentages massiques. Le rendement r est donné par la relation :

$$r = (Ci\text{-}Ce) / Ci \times 100(\%)$$

où Ci représente la concentration initiale en polymère fonctionnalisé et Ce la concentration de la solution après mise en contact de la solution avec les particules solides et séparation des particules solides par centrifugation.

Le taux d'adsorption est donné par

$$t = [(Ci\text{-}Ce)m_s] / m_c S$$

où $m_s$ est la masse de solution et $m_c$ la masse de $TiO_2$ de surface spécifique S.

Dans le cas présent :

$m_s = 10g$
$S = 10\ m^2 / g$
$m_c = 3\ g$

Le tracé de la courbe de la figure 1 donnant le taux d'adsorption en fonction de la concentration initiale Ci en polymère fonctionnalisé à 20°C (isotherme d'adsorption) montre clairement l'existence d'un palier dont on détermine le début par tracé des tangentes comme indiqué sur la figure 1.

Dans le cas particulier de cet exemple, le début du palier lu sur la courbe correspond à une concentration initiale en polymère fonctionnalisé de 1,8 % en masse, ce qui correspond d'après la formule ci-dessus à 60 mg de polymère pour 1 g de $TiO_2$.

La dispersibilité est évaluée à partir de mesures de tailles de particules à l'aide du COULTER LS130.

Les figures 2a à 2d donnent les analyses granulométriques du $TiO_2$ correspond respectivement à des valeurs de Ci de 0, 1, 2 et 3 % pour le même exemple que l'isotherme d'adsorption. Le polymère fonctionnalisé permet de réduire de façon considérable les agrégats jusqu'à les faire disparaître lorsque le recouvrement du $TiO_2$ est total (c'est-à-dire pour des concentrations initiales en polymère correspondant au palier de l'isotherme).

Pour ce type d'exemple, un très bon dispersant doit réduire la quantité d'agrégats à une valeur inférieure à 1 % ou au moins réduire considérablement leur taille.

La stabilisation est déterminée par la variation de l'adsorbance du surnageant en fonction de la durée d'une centrifugation de 120 rpm/mn2 (Shimadzu SA-CP3).

La figure 3 donne l'absorbance des suspensions en fonction du temps respectivement pour les suspensions de $TiO_2$ non traité (Ci = 0 %) ainsi que pour des concentrations Ci de 1 % (avant le palier de l'isotherme) et 3 % (sur le palier de l'isotherme).

Lorsqu'il y a décantation au cours du temps, le dépôt formé reste facilement redispersible dans le milieu après une agitation plus ou moins longue. Le polymère recouvrant totalement la surface empêchant l'agrégation du pigment.

En conclusion, il faut 60 mg de polymère fonctionnalisé pour disperser et stabiliser 1 g de $TiO_2$.

Exemple 2 : polyméthacrylate de méthyle fonctionnalisé par la glycine 2.a.Synthèse

- 10 g de glycine sont solubilisés dans 39 g d'eau,
- 136 g d'acide acétique sont ensuite versés progressivement.

La solution obtenue est placée dans un réacteur similaire à celui utilisé dans l'exemple 1.a.

- 0,16 g d'AIBN sont solubilisés dans 10 g de MMA.

Le tout est ajouté à la solution précédente.

L'ensemble est chauffé à 60°C durant 3 h. On observe une précipitation partielle du polymère en cours de réaction.

Le produit précipité et celui resté en solution sont recueillis séparément.

Ils sont précipités dans le n-butanol. Séchés, éventuellement lavés à l'eau, ils sont ensuite solubilisés dans l'acétone et reprécipités dans l'eau.

Leur masse moléculaire en nombre est déterminée par GPC.

- Pour le polymère précipité lors de la polymérisation, Mn = 133.300.
- Pour le polymère resté en solution lors de la polymérisation, Mn = 54.600.

2.b. Etude de la dispersion d'une poudre de $TiO_2$ dans l'acétate de butyle en présence de PMMA fonctionnalisé par la glycine.

On étudie, comme dans l'exemple précédent, l'influence des polymères fonctionnalisés obtenus selon l'exemple 2.a sur la dispersion de la même poudre d'oxyde de titane dans l'acétate de butyle.

La figure 4 donne les taux d'adsorption et rendement d'adsorption en fonction de la concentration initiale Ci en polymère fonctionnalisé dans le cas de PMMA fonctionnalisé par la glycine de masse molaire moyenne en nombre Mn = 133.300 obtenu selon l'exemple 2.a.

Il apparaît sur la figure 4 que le palier de l'isotherme d'adsorption commence pour des concentrations Ci = 1,9 %. Le calcul permet donc d'établir que 63 mg de polymère fonctionnalisé dans le cas présent sont adsorbés par 1 g de $TiO_2$.

La dispersibilité de la même poudre de $TiO_2$ dans l'acétate de butyle en présence de différentes concentrations du même polymère fonctionnalisé (Mn = 133.300) a été réalisée par mesure de taille des particules au COULTER LS130.

Les résultats pour des concentrations initiales Ci de 0 ($TiO_2$ brut), 1 %, 2 %, 3 % sont donnés respectivement sur les courbes 5a, 5b, 5c, 5d, qui montrent clairement que le polymère fonctionnalisé permet de réduire considérablement la taille des agrégats jusqu'à les faire disparaître lorsque les concentrations initiales en polymère correspondent au palier de l'isotherme (à partir de 1,9 % dans le cas présent).

La figure 6 qui donne la variation de l'absorbance des suspensions de $TiO_2$ non traité ainsi que pour des concentrations Ci de 1 % (avant le palier de l'isotherme) et 3 % (sur le palier de l'isotherme) en PMMA fonctionnalisé par la glycine de masse molaire moyenne en nombre de 133.300 montre clairement l'effet de ce polymère pour stabiliser la dispersion de $TiO_2$ dans l'acétate de butyle.

Dans le cas du PMMA fonctionnalisé par la glycine de Mn = 133.300, il faut donc 63 mg pour disperser et stabiliser environ 1 g de $TiO_2$ en milieu acétate de butyle.

La figure 7 établie de façon analogue à la courbe 6 mais pour le PMMA fonctionnalisé par la glycine de Mn = 54.600 montre également très clairement l'effet stabilisant de ce produit à une concentration initiale Ci de 4,8 %.

Exemple 3 : Polyméthacrylate de méthyle non fonctionnalisé (comparatif) 3.a. Synthèse

0,32 g d'AIBN et 20 g de méthacrylate de méthyle sont solubilisés dans un mélange contenant 136 g d'acide acétique et 39 g d'eau.

La solution obtenue placée dans un réacteur double enveloppe équipé d'une ancre d'agitation, d'un réfrigérant et d'une circulation d'azote et est chauffée à 60°C durant 6 h.

Le mélange recueilli est précipité dans le n-butanol afin d'éliminer les restes de MMA, d'AIBN et de ses produits de décomposition.

La masse moléculaire en nombre du polymère, déterminée par GPC, est Mn = 92.400.

3.b. Effet de PMMA non fonctionnalisé sur la dispersion d'une poudre de $TiO_2$ en milieu acétate de butyle.

On étudie à titre de comparaison l'effet de polymère non fonctionnalisé obtenu dans l'exemple 3a sur la dispersion de la même poudre de $TiO_2$ en milieu acétate de butyle.

La courbe représentée à la figure 8 représente les variations de l'isotherme et du rendement d'adsorption en fonction de la concentration initiale Ci en polymère PMMA non fonctionnalisé. Cette courbe, comparée aux courbes représentées aux figures 1 et 4 pour les PMMA fonctionnalisés selon l'invention, montre que le palier de l'isotherme est situé à une valeur plus faible, correspondant à une valeur de Ci d'environ 1 %.

Les figures 9a, 9b et 9c qui donnent les résultats obtenus par mesure au COULTER LS130 de la taille des particules respectivement pour des concentrations Ci en polymère de 0, 1 % et 3 % montrent clairement qu'en présence de polymère non fonctionnalisé il n'y, a nullement dispersion mais au contraire plutôt agrégation des particules.

Les courbes d'absorbance de la suspension en fonction du temps établies pour des concentrations initiales Ci en polymère de 0 %, 1 % et 3 % montrent également qu'il y a plutôt destabilisation de la suspension en présence de polymère non fonctionnalisé et non stabilisation.

Exemple 4 : applications dans le domaine cosmétique - vernis à ongles

Dans les exemples ci-après, les pourcentages sont indiqués en poids, sauf indications contraires.

Selon une méthode de l'art antérieur, bien connue de l'homme de l'art, on prépare les vernis à ongles à partir de "solutions colorantes" de différentes teintes, que l'on mélange avec une base pour vernis à ongles.

Ces "solutions colorantes" sont en fait des dispersions de pigments dans une base contenant de la nitrocellulose, cette base pouvant être la même que celle utilisée pour la formulation finale du vernis. De préférence, les pigments

sont préalablement broyés dans un solvant, tel que l'acétate de butyle, au moyen d'un broyeur approprié tel que, par exemple, un broyeur à billes de type Dyno-mill.

Suivant la présente invention, le broyage du pigment est réalisé en présence du polymère fonctionnalisé selon l'invention, utilisé comme agent facilitant la dispersion du pigment dans le solvant.

De préférence, la proportion de polymère utilisé est de l'ordre de 2 à 7 % par rapport au poids du pigment. De préférence encore, cette proportion est de 5 % environ.

Ainsi, on a préparé les broyés suivants:

- broyé n°1 :
  . Oxyde de fer noir $Fe_3O_4$ 50 %
  . Polymère fonctionnalisé de Mn = 87500 obtenu selon l'exemple 1 2,5 %
  . Acétate de butyle qsp 100 %
- broyé n°2 :
  . Oxyde de titane $TiO_2$ 70 %
  . Polymère fonctionnalisé de Mn = 87500 obtenu selon l'exemple 1 2,8 %
  . Acétate de butyle qsp 100 %
- broyé n°3 :
  . Pigment organique rouge DC Red 7 25 %
  . Polymère fonctionnalisé de Mn = 87500 obtenu selon l'exemple 1 1,25 %
  . Acétate de butyle qsp 100 %

Comme cela a été exposé plus haut, les broyés sont incorporés à une base nitrocellulosique "diluante" pour préparer différentes solutions colorantes, chacune ayant sa propre teinte selon la nature et la concentration du pigment qu'elle contient.

Par exemple, la composition de la base "diluante" est la suivante :

| | | |
|---|---|---|
| - nitrocellulose | 10 à 30 %, | par exemple 15 % |
| - Lustralite®(arylsulfonamide) | 8 à 15 %, | par exemple 10 % |
| - dibutyle-phtalate | 4 à 7 %, | par exemple 5 % |
| - Néocryl®(résine acrylique) | 0 à 5 %, | par exemple 2 % |
| - acétate de butyle | 5 à 50 %, | par exemple 20 % |
| - acétate d'éthyle | 5 à 50 %, | par exemple 20 % |
| - bentonite | 0,8 à 1,5 %, | par exemple 1 % |
| - toluène | 0 à 30 %, | par exemple 25 % |
| - isopropanol | 1 à 5 %, | par exemple 2 % |
| | 100 % | 100 % |

La quantité de broyé introduite dans la base diluante est telle que la concentration en pigment dans la solution colorante est généralement inférieure ou égale à 20 % environ.

Suivant la teinte souhaitée de la composition finale de vernis à ongles, on introduit dans une base, telle que la base ci-dessus, différentes solutions colorantes à différentes concentrations. La teneur en pigment du vernis final est en général de l'ordre de 2 à 4 %.

## Revendications

**1.** Polymère fonctionnalisé en bout de chaîne répondant à la formule :

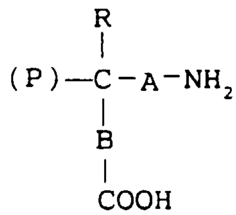 (1)

dans laquelle :

- la chaîne polymérique (P) est une chaîne hydrophobe obtenue par polymérisation radicalaire d'au moins un monomère,
- R représente un atome d'hydrogène ou une chaîne hydrocarbonée comprenant 1 à 8 atomes de carbone, linéaire ou ramifiée, éventuellement substituée par au moins un groupe choisi parmi $CO_2H$, $NH_2$, OH ou un groupe phényle, lui-même éventuellement substitué,
- A et B, identiques ou différents, représentent chacun une liaison simple, une chaîne hydrocarbonée saturée ou insaturée, linéaire ou ramifiée comprenant de 1 à 16 atomes de carbone, pouvant contenir une fonction amide, une chaîne peptidique, comprenant 2 à 4 acides aminés, en particulier, naturels,
- les groupements COOH et/ou $NH_2$ étant libres ou salifiés.

**2.** Polymère fonctionnalisé selon la revendication 1, caractérisé en ce que la chaîne polymérique (P) a une masse molaire moyenne en nombre comprise entre 500 et 250.000.

**3.** Polymère fonctionnalisé selon la revendication 1 ou 2, caractérisé en ce qu'il répond à la formule:

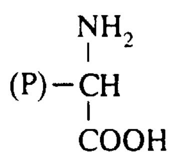 (3)

dans laquelle :
(P) est une chaîne polymérique telle que définie à la revendication 1 ou 2.

**4.** Polymère fonctionnalisé selon la revendication 1 ou 2, caractérisé en ce qu'il répond à la formule:

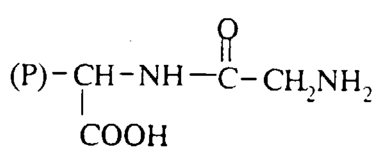 (4)

dans laquelle :
(P) est une chaîne polymérique telle que définie à la revendication 1 ou 2.

**5.** Polymère fonctionnalisé selon la revendication 1, caractérisé en ce qu'il répond à la formule :

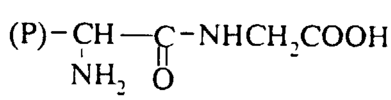 (5)

dans laquelle :
(P) est une chaîne polymérique telle que définie à la revendication 1 ou 2.

**6.** Polymère fonctionnalisé selon la revendication 1 ou 2, caractérisé en ce qu'il répond à la formule :

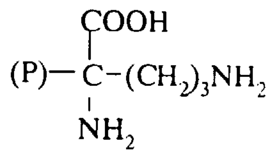

(6)

dans laquelle :
(P) est une chaîne polymérique telle que définie à la revendication 1 ou 2.

**7.** Polymère fonctionnalisé selon la revendication 1 ou 2, caractérisé en ce qu'il répond à la formule :

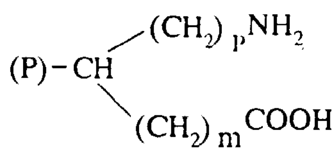

(7)

dans laquelle :
(P) est une chaîne polymérique telle que définie à la revendication 1 ou 2, m et p sont des entiers compris entre 0 et 11 et dont la somme est comprise entre 2 et 11.

**8.** Polymère fonctionnalisé selon l'une des revendications 1 à 7, caractérisé en ce qu'il est obtenu par polymérisation radicalaire d'au moins un monomère en présence d'un acide aminé ou d'un dérivé d'acide aminé répondant à la formule :

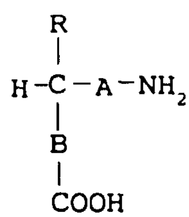

(2)

dans laquelle R, A et B ont les significations données dans la revendication 1.

**9.** Polymère fonctionnalisé selon la revendication 7, caractérisé en ce qu'il est obtenu dans un procédé comprenant :

- une première étape de polymérisation radicalaire d'au moins un monomère conduisant à une chaîne polymérique hydrophobe P, en présence d'un lactame agissant comme agent de transfert de chaîne et répondant à la formule :

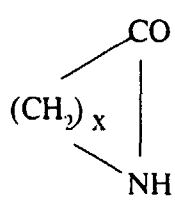

(8)

dans laquelle x est un entier compris entre 3 et 12,
- suivie d'une deuxième étape d'hydrolyse du produit obtenu lors de la première étape.

**10.** Polymère fonctionnalisé selon l'une des revendications 1 à 9, caractérisé en ce que ledit monomère est un monomère acrylique ou vinylique.

**11.** Polymère fonctionnalisé selon la revendication 10, caractérisé en ce que ledit monomère est un monomère acrylique choisi dans le groupe constitué des acrylates, méthacrylates, éthylacrylates d'un groupe hydrocarboné en C1 à C18, saturé ou insaturé, en particulier allylique, linéaire, ramifié ou comprenant un cycle.

**12.** Polymère fonctionnalisé selon l'une des revendications 1 à 11, caractérisé en ce que ladite chaîne polymérique (P) résulte de la polymérisation radicalaire du méthacrylate de méthyle.

**13.** Produit intermédiaire répondant à la formule :

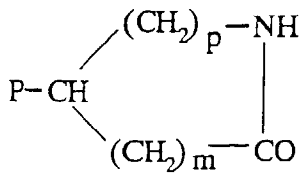

(9)

dans laquelle :

- (P) est une chaîne polymérique hydrophobe résultant de la polymérisation radicalaire d'un monomère acrylique ou vinylique,
- p et m sont des entiers compris entre 0 et 11 et dont la somme est comprise entre 2 et 11.

**14.** Utilisation d'un polymère fonctionnalisé selon l'une des revendications 1 à 12 et dans lequel la chaîne polymérique (P) est une chaîne hydrophobe obtenue par polymérisation radicalaire d'au moins un monomère et dont la masse molaire moyenne en nombre est comprise entre 500 et 250.000, comme agent tensioactif.

**15.** Utilisation selon la revendication 14, caractérisée en ce que ledit polymère fonctionnalisé est utilisé comme agent mouillant d'une surface solide ou d'une particule solide.

**16.** Utilisation selon la revendication 14, caractérisée en ce que ledit polymère fonctionnalisé est utilisé comme agent dispersant de particules solides dans un milieu organique.

**17.** Utilisation selon la revendication 14, caractérisée en ce que ledit polymère fonctionnalisé a une masse molaire moyenne en nombre supérieure à 1 000, de préférence comprise entre 5 000 et 150 000 et est utilisé comme agent stabilisant de dispersions de particules solides dans un milieu organique.

**18.** Utilisation selon l'une des revendications 15 à 17, caractérisée en ce que lesdites particules sont des particules d'oxyde métallique, par exemple $TiO_2$, $SiO_2$, $Al_2O_3$, des oxydes de fer, tels que goethite, hématite, magnétite, d'oxyde métallique recouvert de molécules de colorants organiques, ou des particules organiques, par exemple du type rosinate, coprécipitées avec un colorant organique.

**19.** Utilisation comme agent tensioactif selon la revendication 14 d'un polymère fonctionnalisé tel que défini dans la revendication 14, de masse molaire moyenne en nombre de préférence inférieure à 20000, de préférence comprise entre 500 et 10 000, pour la préparation d'une solution micellaire ou d'une microdispersion de polymères, en particulier un microgel ou un microlatex.

**20.** Utilisation d'un polymère fonctionnalisé selon l'une des revendications 1 à 12 et dans lequel la chaîne polymérique (P) est une chaîne hydrophobe obtenue par polymérisation radicalaire d'au moins un monomère et dont la masse molaire moyenne en nombre est comprise entre 500 et 250.000 pour la préparation d'une composition, en particulier d'une composition de type peinture ou d'une composition alimentaire, phytosanitaire ou cosmétique.

**21.** Utilisation selon la revendication 20, caractérisée en ce que la composition contient des particules solides en suspension, en particulier des pigments, et en ce que ledit polymère fonctionnalisé est mis en présence desdites particules solides, notamment pigments, pour faciliter leur dispersion dans la composition.

**22.** Utilisation selon la revendication 21, caractérisée en ce que la quantité de polymère fonctionnalisé utilisé est comprise entre 2 % et 7 % en poids par rapport au poids total des particules solides dispersées dans la composition.

**23.** Utilisation selon la revendication 20, caractérisée en ce que ledit polymère fonctionnalisé est compris dans une microdispersion de polymères, en particulier un microgel ou un microlatex.

**24.** Utilisation selon la revendication 23, caractérisée en ce que la quantité de microdispersion de polymères, de microgels ou de microlatex est comprise entre 1 % et 20 % en poids par rapport au poids total de la composition.

**25.** Utilisation selon l'une quelconque des revendications 20 à 24, caractérisée en ce que la composition est un vernis à ongles.

**26.** Composition cosmétique, en particulier destinée au soin ou au maquillage des ongles, caractérisée en ce qu'elle contient un polymère fonctionnalisé selon l'une des revendications 1 à 12 et dans lequel la chaîne polymérique (P) est une chaîne hydrophobe obtenue par polymérisation radicalaire d'au moins un monomère et dont la masse molaire moyenne en nombre est comprise entre 500 et 250.000.

**27.** Composition cosmétique selon la revendication 26, caractérisée en ce que le polymère fonctionnalisé est compris dans une microdispersion de polymères, en particulier dans un microgel ou un microlatex.

**28.** Composition cosmétique selon l'une des revendications 26 ou 27, caractérisée en ce qu'elle contient en outre de la nitrocellulose.

## Claims

**1.** A chain end functionalised polymer of formula :

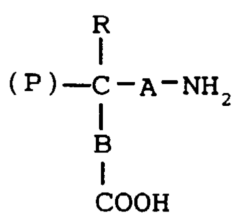

(1)

in which:

- the polymer chain (P) is a hydrophobic chain obtained by radical polymerisation of at least one monomer,
- R represents a hydrogen atom or a linear or branched hydrocarbon chain having 1 to 8 carbon atoms optionally substituted with at least one group selected from $CO_2H$, $NH_2$, OH or a phenyl group, itself being optionally substituted,
- A and B, identical or different, each represent a single bond, a saturated or unsaturated linear or branched hydrocarbon chain having from 1 to 16 carbon atoms, it being possible for it to contain an amide bond or a peptide chain having 2 to 4 amino acids, particularly natural amino acids,
- the COOH and/or $NH_2$ groups being free or salified.

**2.** The functionalised polymer according to claim 1, characterised in that the polymer chain (P) has a number average molar mass between 500 and 250,000.

**3.** The functionalised polymer according to claim 1 or 2, characterised in that it is of formula:

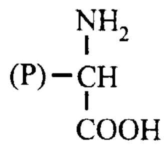

(3)

in which:
(P) is a polymer chain such as defined in claim 1 or 2.

**4.** The functionalised polymer according to claim 1 or 2, characterised in that it is of formula :

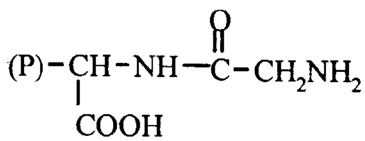

(4)

in which:
(P) is a polymer chain such as defined in claim 1 or 2.

**5.** The functionalised polymer according to claim 1, characterised in that it is of formula:

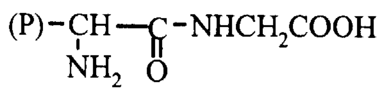

(5)

in which:
(P) is a polymer chain such as defined in claim 1 or 2.

**6.** The functionalised polymer according to claim 1 or 2, characterised in that it is of formula:

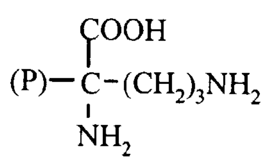

(6)

in which:
(P) is a polymer chain such as defined in claim 1 or 2.

**7.** The functionalised polymer according to 1 or 2, characterised in that it is of formula:

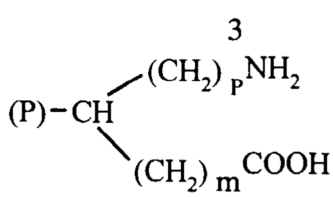

(7)

in which:
(P) is a polymer chain such as defined in claim 1 or 2,
m and p are integers between 0 and 11 and whose sum is between 2 and 11.

**8.** The functionalised polymer according to one of claims 1 to 7, characterised in that it is obtained by radical polymerisation of at least one monomer in the presence of an amino acid of the formula:

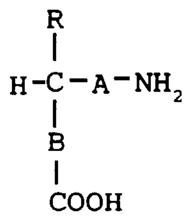

(2)

in which R, A and B have the meanings given in claim 1.

**9.** The functionalised polymer according to claim 7, characterised in that it is obtained in a method comprising :

- a first step of radical polymerisation of at least one monomer leading to a hydrophobic polymer chain P in the presence of a lactam acting as chain transfer agent and being of formula :

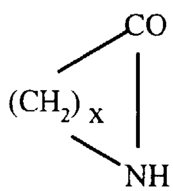

(8)

  in which x is an integer between 3 and 12,
- followed by a second hydrolysis step of the product obtained during the first step.

**10.** The functionalised polymer according to one of claims 1 to 9, characterised in that said monomer is an acrylic or vinylic monomer.

**11.** The functionalised polymer according to claim 10, characterised in that said monomer is an acrylic monomer selected from the group consisting of the acrylates, methacrylates, ethylacrylates of a saturated or unsaturated, particularly allylic, linear, branched or ring-containing, C1 to C18 hydrocarbon group.

**12.** The functionalised polymer according to one of claims 1 to 11, characterised in that said polymer chain (P) results from the radical polymerisation of methyl methacrylate.

**13.** An intermediate product of the formula:

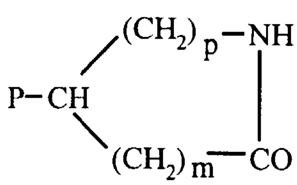

(9)

in which :

- (P) is a hydrophobic polymer chain resulting from the radical polymerisation of an acrylic or vinylic monomer,
- p and m are integers between 0 and 11 and whose sum is between 2 and 11.

**14.** Use of a functionalised polymer according to one of claims 1 to 12 and in which the polymer chain (P) is a hydrophobic chain obtained by radical polymerisation of at least one monomer and whose number average molar mass is between 500 and 250,000, as a surfactant.

**15.** Use according to claim 14, characterised in that said functionalised polymer is used as a wetting agent of a solid surface or solid particle.

**16.** Use according to claim 14, characterised in that said functionalised polymer is used as a dispersing agent of solid particles in an organic medium.

**17.** Use according to claim 14, characterised in that said functionalised polymer has a number average molecular mass greater than 1,000, preferably between 5,000 and 150,000 and is used as a solid particle dispersion stabilising agent in an organic medium.

**18.** Use according to one of claims 15 to 17, characterised in that said particles are metal oxide particles, for example $TiO_2$, $SiO_2$, $Al_2O_3$, iron oxides, such as goethite, haematite, magnetite, a metal oxide covered with organic colorant molecules, or organic particles, for example of the rosinate type, coprecipitated with an organic colorant.

**19.** Use, as a surfactant according to claim 14, of a functionalised polymer such as defined in claim 14, of number average molar mass preferably lower than 20,000, preferably between 500 and 10,000, for the preparation of a micellar solution or a microdispersion of polymers, particularly a microgel or a microlatex.

**20.** Use of a functionalised polymer according to one of claims 1 to 12 and in which the polymer chain (P) is a hydrophobic chain obtained by radical polymerisation of at least one monomer and whose number average molar mass is between 500 and 250,000 for the preparation of a composition, in particular a composition of the paint type or a food, phytosanitary or cosmetic composition.

**21.** Use according to claim 20, characterised in that the composition contains solid particles in suspension, pigments in particular, and that said functionalised polymer is placed in the presence of said solid particles, especially pigments, in order to facilitate their dispersion in the composition.

**22.** Use according to claim 21, characterised in that the amount of functionalised polymer used is between 2 % and 7 % by weight based on the total weight of the solid particles dispersed in the composition.

**23.** Use according to claim 20, characterised in that said functionalised polymer is comprised in a microdispersion of polymers, in particular a microgel or a microlatex.

**24.** Use according to claim 23, characterised in that the amount of microdispersion of polymers, of microgels or of microlatex is between 1 % and 20 % by weight based on the total weight of the composition.

**25.** Use according to any one of claims 20 to 24, characterised in that the composition is a nail varnish.

**26.** A cosmetic composition, particularly intended for the care or the make-up of the nails, characterised in that it contains a functionalised polymer according to one of claims 1 to 12 and in which the polymer chain (P) is a hydrophobic chain obtained by radical polymerisation of at least one monomer and whose number average molar mass is between 500 and 250,000.

**27.** The cosmetic composition according to claim 26, characterised in that the functionalised polymer is comprised in a microdispersion of polymers, in particular a microgel or a microlatex.

**28.** The cosmetic composition according to one of claims 26 or 27, characterised in that it further contains nitrocellulose.

## Patentansprüche

1. Am Kettenende funktionalisiertes Polymer der Formel:

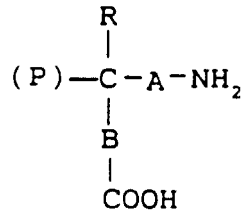

(1),

worin:

- die Polymer-Kette (P) eine hydrophobe Kette ist, die durch radikalische Polymerisation zumindest eines Monomers erhalten wird,
- R ein Wasserstoffatom oder eine Kohlenwasserstoff-Kette mit 1 bis 8 Kohlenstoffatomen, linear oder verzweigt, gegebenenfalls substituiert durch zumindest eine Gruppe ausgewählt aus $CO_2H$, $NH_2$, OH oder einer Phenyl-Gruppe, die gegebenenfalls selbst substituiert ist, bedeutet,
- A und B, die gleich oder verschieden sind, jeweils eine Einfachbindung, eine gesättigte oder ungesättigte, lineare oder verzweigte Kohlenwasserstoff-Kette mit 1 bis 16 Kohlenstoffatomen, die eine Amid-Funktion enthalten kann, eine Peptid-Kette mit 2 bis 4 insbesondere natürlichen Aminosäuren darstellen,
- die Gruppen COOH und/oder $NH_2$ frei oder versalzt sind.

2. Funktionalisiertes Polymer nach Anspruch 1, dadurch gekennzeichnet, daß die Polymer-Kette (P) eine zahlenmittlere Molmasse zwischen 500 und 250 000 aufweist.

3. Funktionalisiertes Polymer nach Anspruch 1 oder 2, dadurch gekennzeichnet, daß es die Formel aufweist:

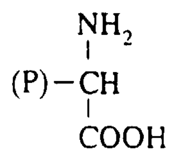

(3),

worin:
(P) eine wie in Anspruch 1 oder 2 definierte Polymer-Kette ist.

4. Funktionalisiertes Polymer nach Anspruch 1 oder 2, dadurch gekennzeichnet, daß es die Formel aufweist:

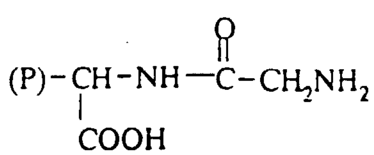

(4),

worin:
(P) eine wie in Anspruch 1 oder 2 definierte Polymer-Kette ist.

5. Funktionalisiertes Polymer nach Anspruch 1, dadurch gekennzeichnet, daß es die Formel aufweist:

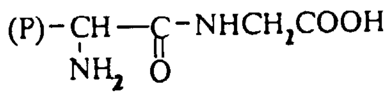 (5),

worin:
(P) eine wie in Anspruch 1 oder 2 definierte Polymer-Kette ist.

**6.** Funktionalisiertes Polymer nach Anspruch 1 oder 2, dadurch gekennzeichnet, daß es die Formel aufweist:

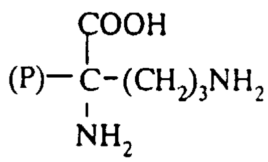 (6),

worin:
(P) eine wie in Anspruch 1 oder 2 definierte Polymer-Kette ist.

**7.** Funktionalisiertes Polymer nach Anspruch 1 oder 2, dadurch gekennzeichnet, daß es die Formel aufweist:

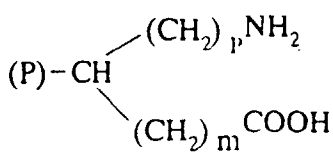 (7),

worin:

(P) eine wie in Anspruch 1 oder 2 definierte Polymer-Kette ist,
m und p ganze Zahlen zwischen Null und 11 sind, deren Summe zwischen 2 und 11 beträgt.

**8.** Funktionalisiertes Polymer nach einem der Ansprüche 1 bis 7, dadurch gekennzeichnet, daß es erhalten wird durch radikalische Polymerisation zumindest eines Monomers in Anwesenheit einer Aminosäure oder eines Aminosäure-Derivats der Formel:

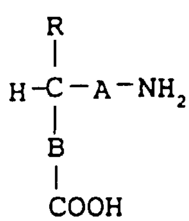 (2),

worin R, A und B die in Anspruch 1 angegebenen Bedeutungen haben.

**9.** Funktionalisiertes Polymer nach Anspruch 7, dadurch gekennzeichnet, daß es durch ein Verfahren erhalten wird, das umfaßt:

- einen erste radikalischen Polymerisationsschritt zumindest eines Monomers, der zu einer hydrophoben Polymer-Kette P führt, in Anwesenheit eines Lactams, das als Kettentransfermittel wirkt und die Formel aufweist:

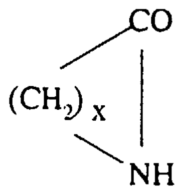

(8),

worin x eine ganze Zahl zwischen 3 und 12 ist,
- gefolgt von einem zweiten Hydrolyseschritt des im ersten Schritt erhaltenen Produkts.

**10.** Funktionalisiertes Polymer nach einem der Ansprüche 1 bis 9, dadurch gekennzeichnet, daß das Monomer ein Acryl- oder Vinyl-Monomer ist.

**11.** Funktionalisiertes Polymer nach Anspruch 10, dadurch gekennzeichnet, daß das Monomer ein Acryl-Monomer ist, ausgewählt aus der Gruppe bestehend aus Acrylaten, Methacrylaten, Ethylacrylaten einer $C_1$-$C_{18}$-Kohlenwasserstoff-Gruppe, die gesättigt oder ungesättigt, insbesondere Allyl, linear, verzweigt ist oder einen Cyclus enthält.

**12.** Funktionalisiertes Polymer nach einem der Ansprüche 1 bis 11, dadurch gekennzeichnet, daß die Polymer-Kette (P) durch die radikalische Polymerisation von Methylmethacrylat erhalten wird.

**13.** Zwischenprodukt der Formel:

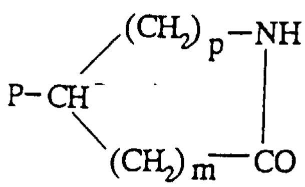

(9),

worin:

- (P) eine hydrophobe Polymer-Kette ist, die durch die radikalische Polymerisation eines Acryl- oder Vinyl-Monomers erhalten wird,
- p und m ganze Zahlen zwischen Null und 11 sind, deren Summe zwischen 2 und 11 beträgt.

**14.** Verwendung eines funktionalisierten Polymers nach einem der Ansprüche 1 bis 12, worin die Polymer-Kette (P) eine hydrophobe Kette ist, die durch radikalische Polymerisation zumindest eines Monomers erhalten wird, und deren zahlenmittlere Molmasse zwischen 500 und 250 000 liegt, als grenzflächenaktiver Stoff.

**15.** Verwendung nach Anspruch 14, dadurch gekennzeichnet, daß das funktionalisierte Polymer als Netzmittel einer festen Oberfläche oder eines festen Teilchens verwendet wird.

**16.** Verwendung nach Anspruch 14, dadurch gekennzeichnet, daß das funktionalisierte Polymer als Dispergiermittel fester Teilchen in einem organischen Medium verwendet wird.

**17.** Verwendung nach Anspruch 14, dadurch gekennzeichnet, daß das funktionalisierte Polymer eine zahlenmittlere Molmasse von mehr als 1000, vorzugsweise zwischen 5000 und 150 000, aufweist und als Stabilisator von Dispersionen fester Teilchen in einem organischen Medium verwendet wird.

**18.** Verwendung nach einem der Ansprüche 15 bis 17, dadurch gekennzeichnet, daß die Teilchen Teilchen aus Metalloxid sind, beispielsweise $TiO_2$, $SiO_2$, $Al_2O_2$, Eisenoxiden, wie Goethit, Hematit, Magnetit, von organischen Farbmittelmolekülen gewonnenem Metalloxid, oder organische Teilchen, beispielsweise vom Rosinat-Typ, die mit einem organischen Farbmittel co-präzipitiert werden.

**19.** Verwendung, als grenzflächenaktiver Stoff nach Anspruch 14, eines funktionalisierten Polymers, das wie in Anspruch 14 definiert ist, mit einer zahlenmittleren Molmasse von vorzugsweise weniger als 20 000, vorzugsweise zwischen 500 und 10 000, bei der Herstellung einer Micellen-Lösung oder einer Mikrodispersion von Polymeren, insbesondere eines Mikrogels oder eines Mikrolatex.

**20.** Verwendung eines funktionalisierten Polymers nach einem der Ansprüche 1 bis 12, worin die Polymer-Kette (P) eine hydrophobe Polymer-Kette ist, die durch radikalische Polymerisation zumindest eines Monomers erhalten wird, und deren zahlenmittlere Molmasse zwischen 500 und 250 000 beträgt, bei der Herstellung einer Zusammensetzung, insbesondere einer Zusammensetzung vom Typ einer Farbe oder einer Nahrungsmittel-, Pflanzenschutz- oder kosmetischen Zusammensetzung.

**21.** Verwendung nach Anspruch 20, dadurch gekennzeichnet, daß die Zusammensetzung suspendierte feste Teilchen, insbesondere Pigmente, enthält, und daß das funktionalisierte Polymer mit den festen Teilchen, insbesondere Pigmenten, in Berührung gebracht wird, um ihre Dispersion in der Zusammensetzung zu erleichtern.

**22.** Verwendung nach Anspruch 21, dadurch gekennzeichnet, daß die Menge des verwendeten funktionalisierten Polymers zwischen 2 Masse-% und 7 Masse-%, bezogen auf die Gesamtmasse der in der Zusammensetzung dispergierten festen Teilchen, beträgt.

**23.** Verwendung nach Anspruch 20, dadurch gekennzeichnet, daß das funktionalisierte Polymer aus einer Mikrodispersion von Polymeren, insbesondere einem Mikrogel oder einem Mikrolatex, besteht.

**24.** Verwendung nach Anspruch 23, dadurch gekennzeichnet, daß die Menge der Polymer-Mikrodispersion, des Mikrogels oder des Mikrolatex zwischen 1 Masse-% und 20 Masse-%, bezogen auf die Gesamtmasse der Zusammensetzung, beträgt.

**25.** Verwendung nach einem der Ansprüche 20 bis 24, dadurch gekennzeichnet, daß die Zusammensetzung ein Nagellack ist.

**26.** Kosmetische Zusammensetzung, insbesondere zum Pflegen oder Schminken der Nägel, dadurch gekennzeichnet, daß sie ein funktionalisiertes Polymer nach einem der Ansprüche 1 bis 12 enthält, worin die Polymer-Kette (P) eine hydrophobe Kette ist, die durch radikalische Polymerisation zumindest eines Monomers erhalten wird, und deren zahlenmittlere Molmasse zwischen 500 und 250 000 beträgt.

**27.** Kosmetische Zusammensetzung nach Anspruch 26, dadurch gekennzeichnet, daß das funktionalisierte Polymer aus einer Mikrodispersion von Polymeren, insbesondere einem Mikrogel oder einem Mikrolatex, besteht.

**28.** Kosmetische Zusammensetzung nach einem der Ansprüche 26 oder 27, dadurch gekennzeichnet, daß sie außerdem Nitrocellulose enthält.

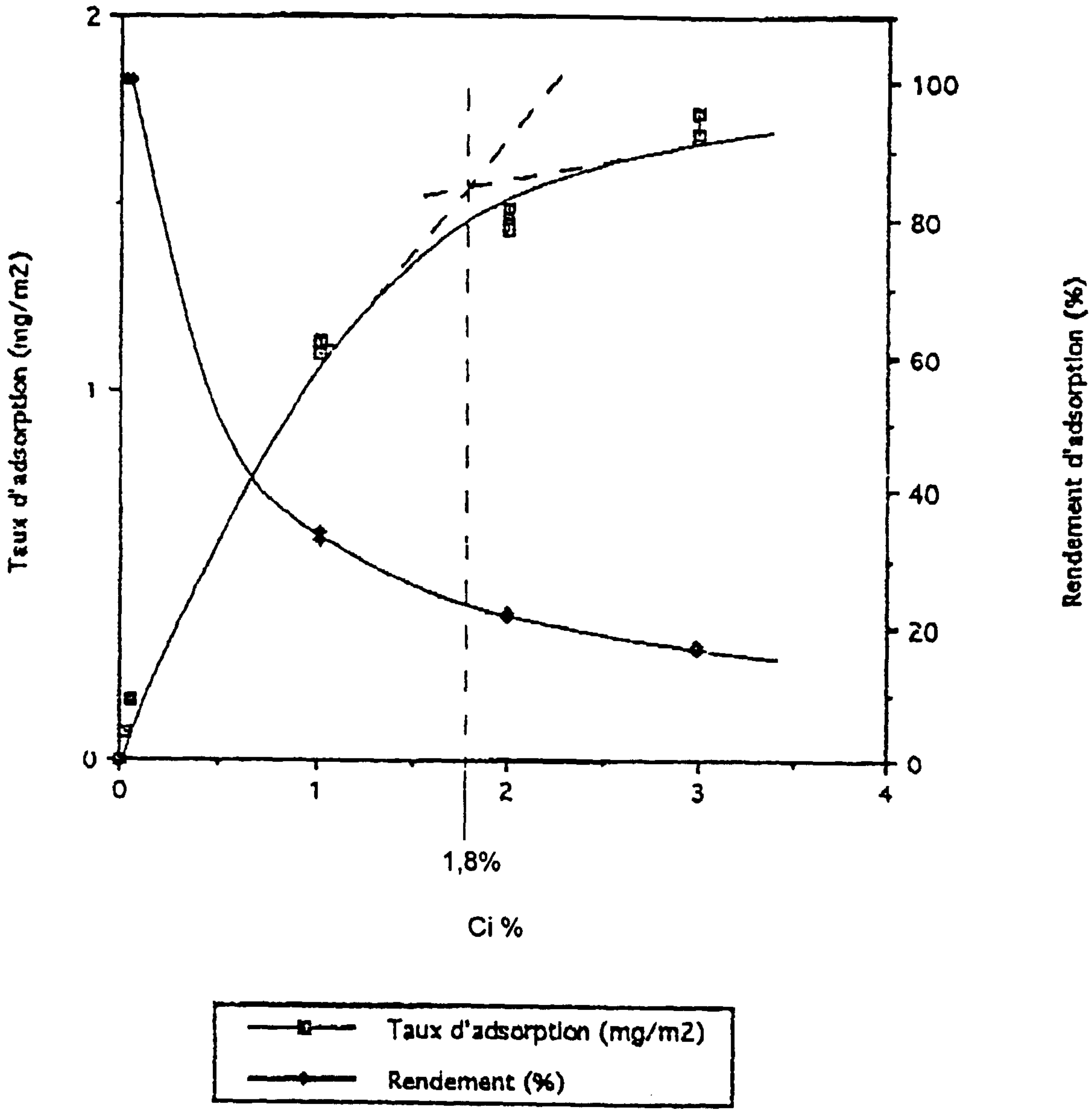
2
1
0
Taux d'adsorption (mg/m2)
100
80
60
40
20
0
Rendement d'adsorption (%)
0
1
2
3
4
1,8%
Ci %
Taux d'adsorption (mg/m2)
Rendement (%)

**FIG.1**

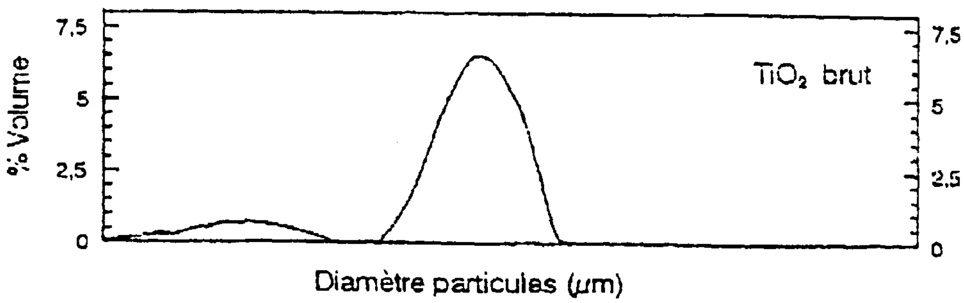


**FIG.2a**

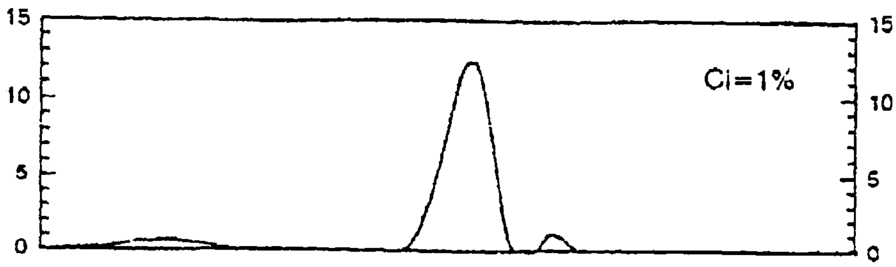


**FIG.2b**

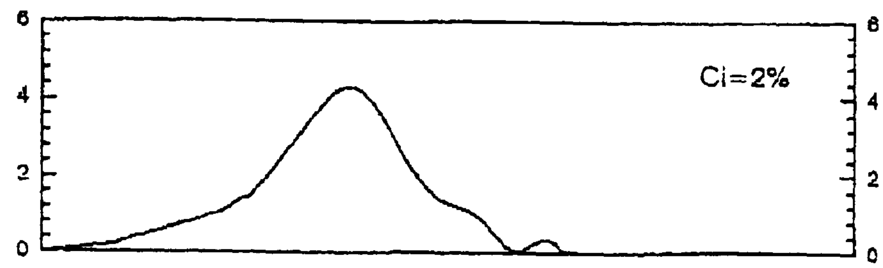


**FIG.2c**

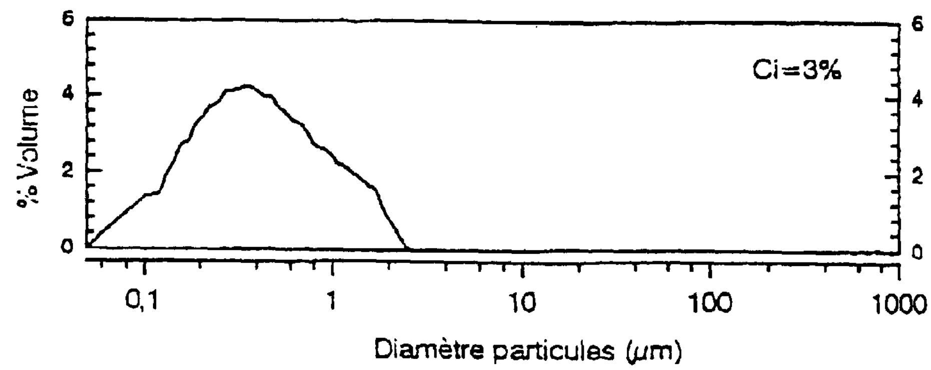


**FIG.2d**

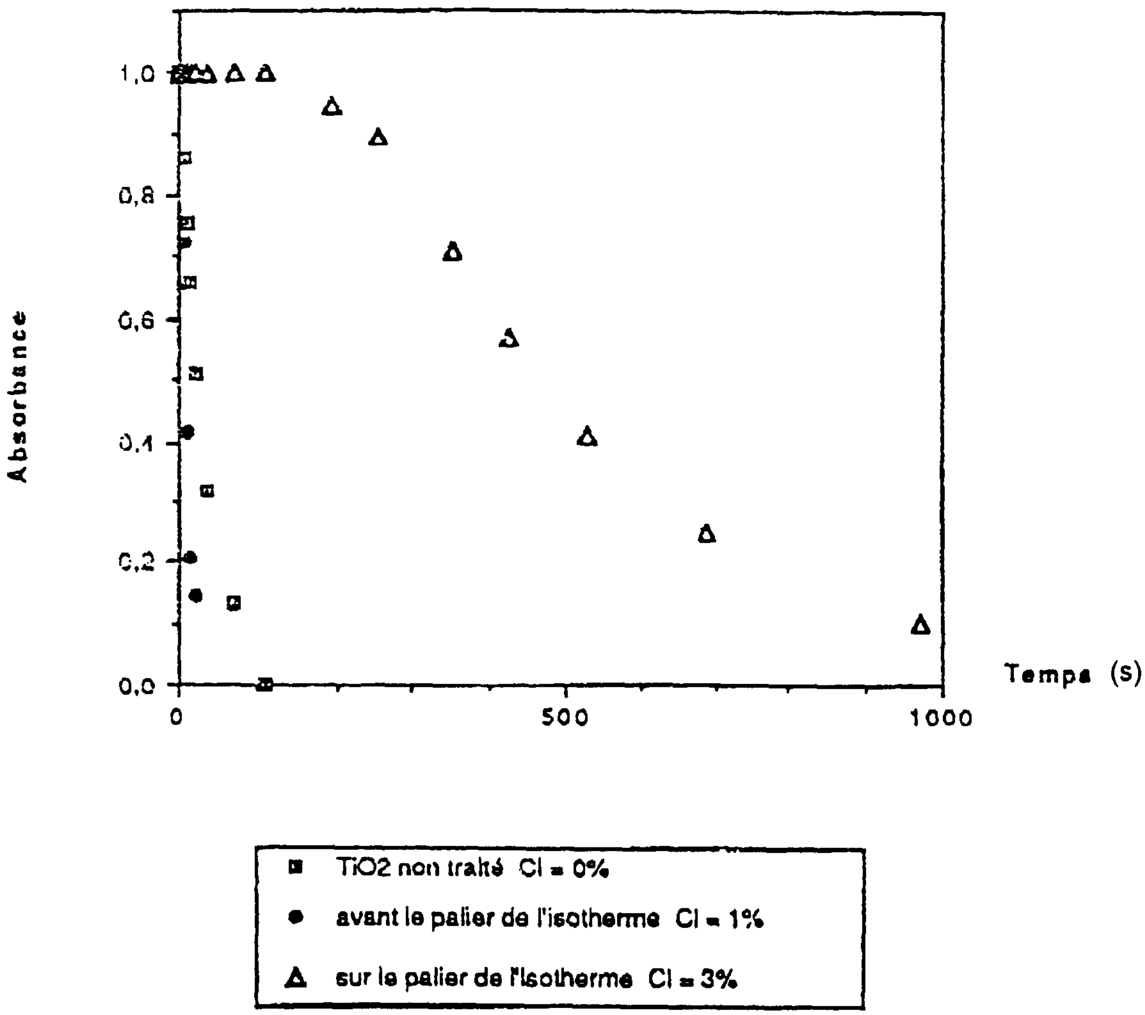
1,0
0,8
0,6
0,4
0,2
0,0
Absorbance
0
500
1000
Temps (s)
TiO2 non traité Cl = 0%
avant le palier de l'isotherme Cl = 1%
sur le palier de l'isotherme Cl = 3%

FIG.3

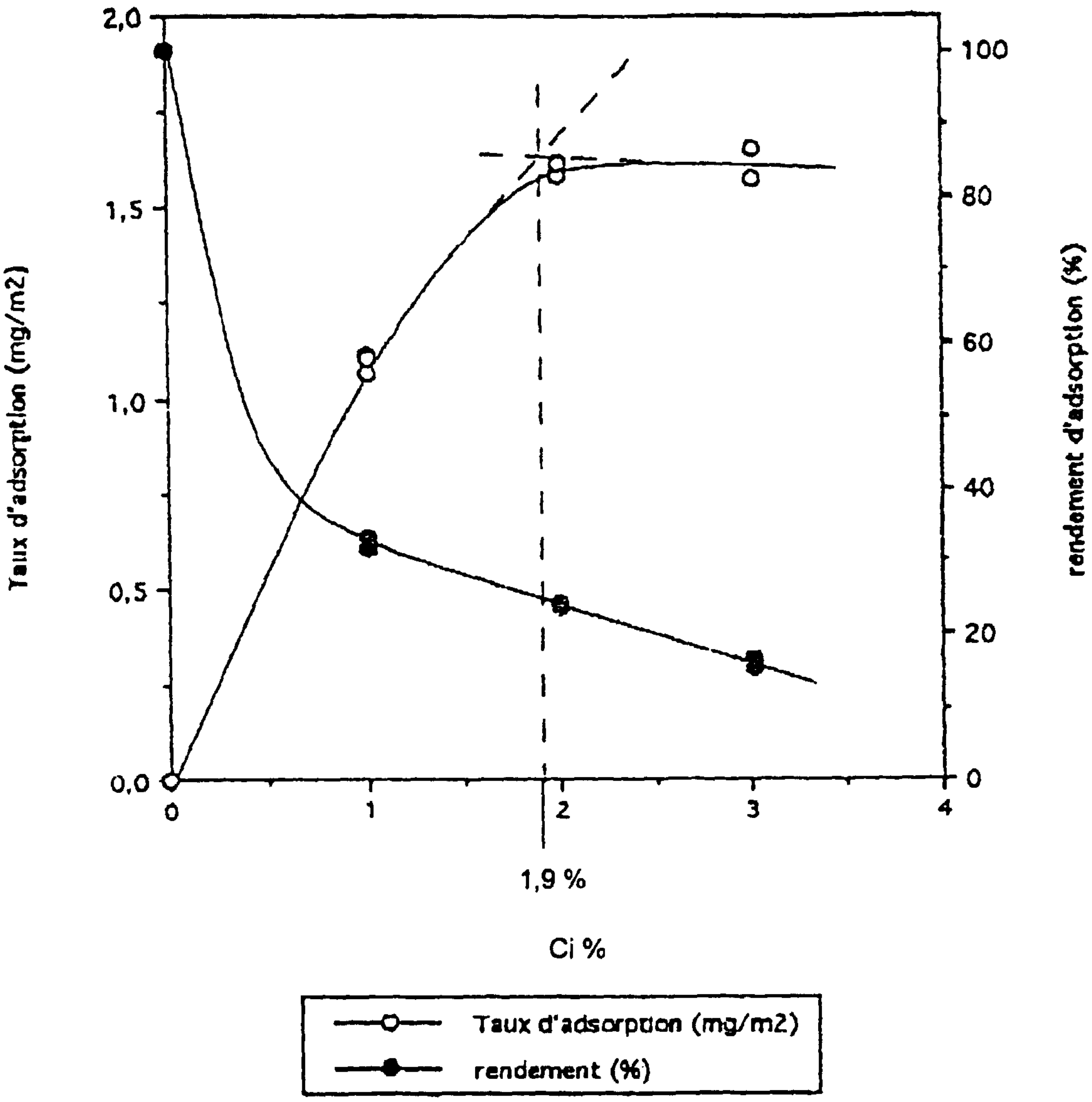
2,0
1,5
1,0
0,5
0,0
Taux d'adsorption (mg/m2)
100
80
60
40
20
0
rendement d'adsorption (%)
0
1
2
3
4
1,9 %
Ci %
Taux d'adsorption (mg/m2)
rendement (%)

**FIG.4**

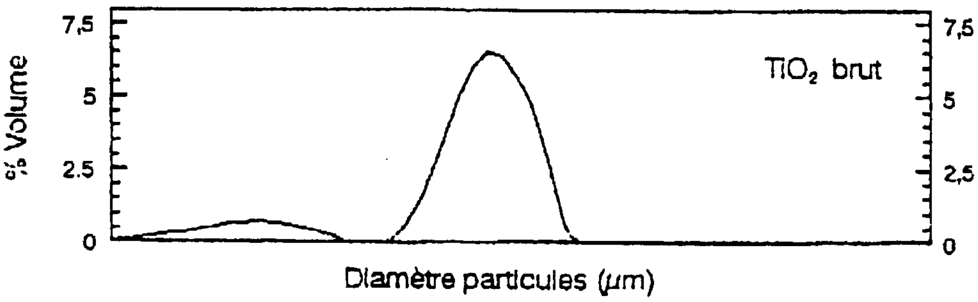


FIG.5a

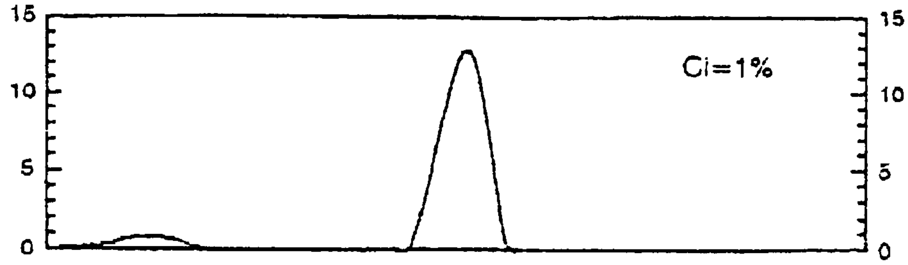


FIG.5b

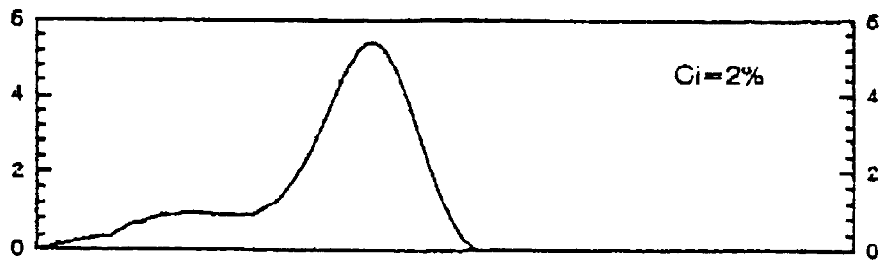


FIG.5c

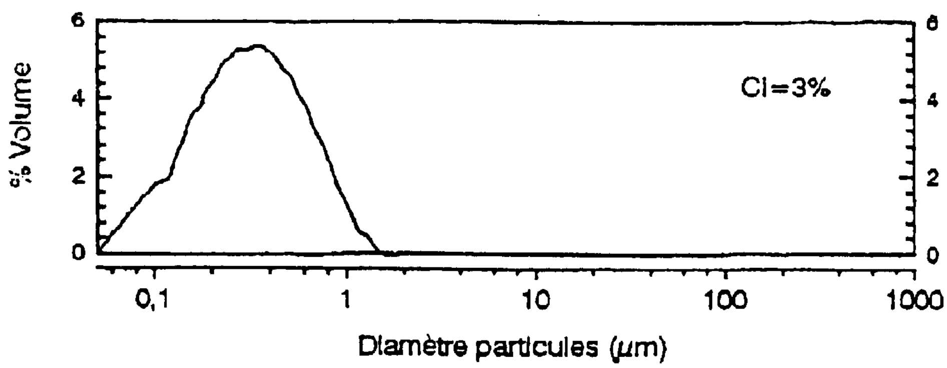


FIG.5d

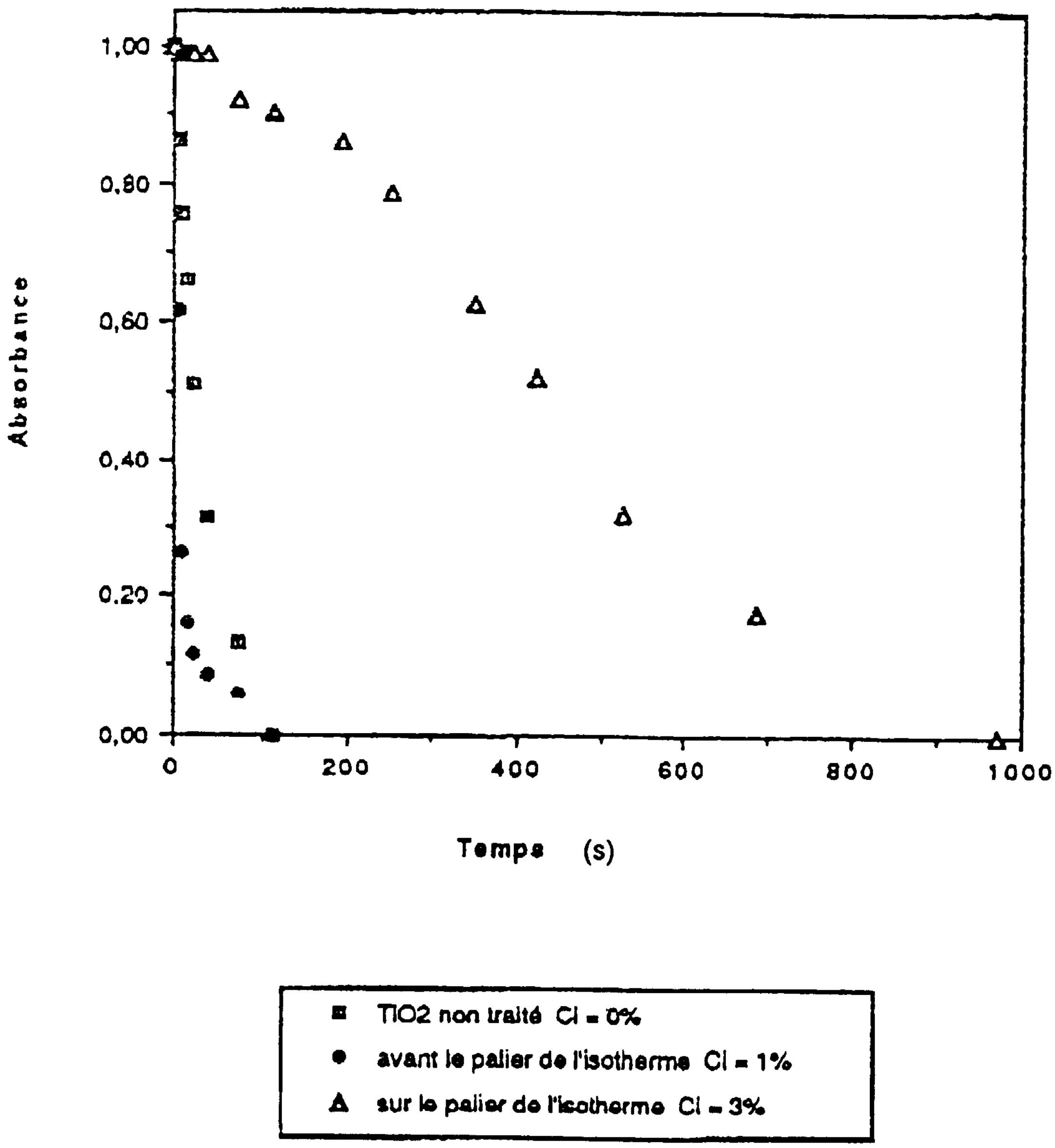
1,00
0,80
0,60
0,40
0,20
0,00
0
200
400
600
800
1000
Absorbance
Temps (s)
TIO2 non traité Cl = 0%
avant le palier de l'isotherme Cl = 1%
sur le palier de l'isotherme Cl = 3%

FIG.6

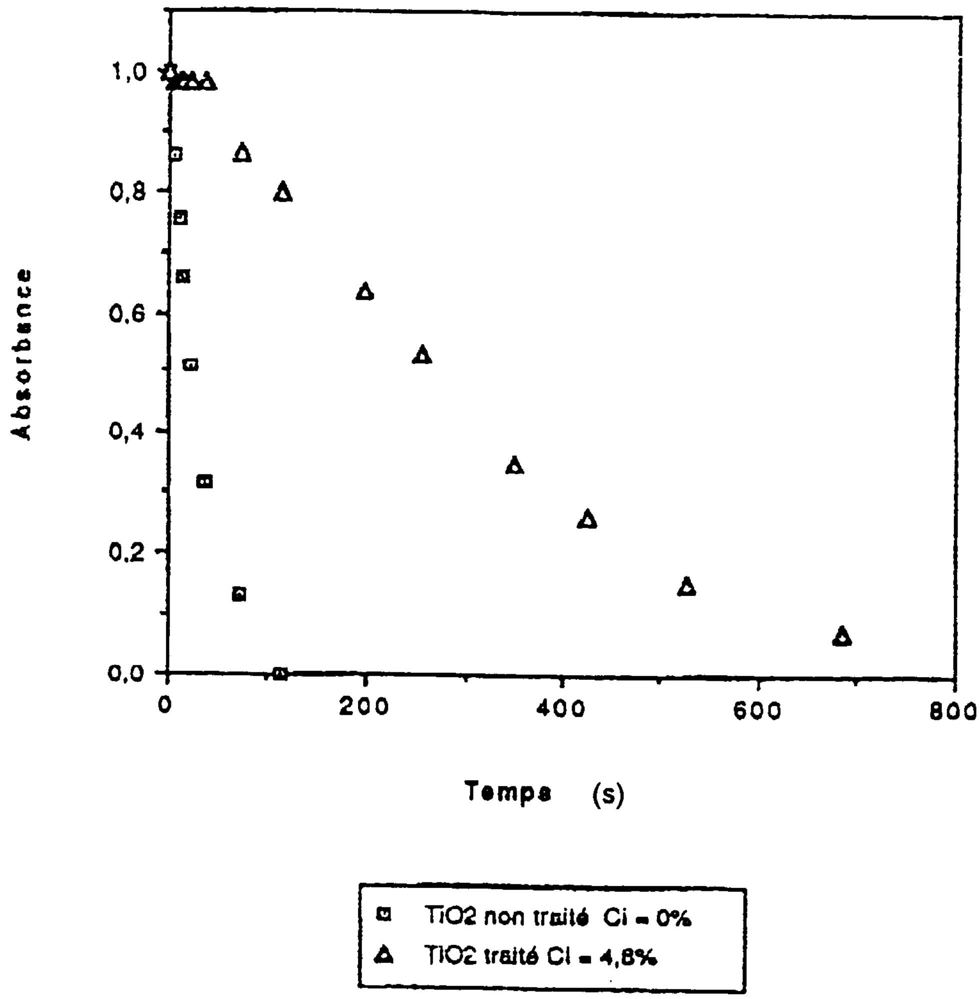
1,0
0,8
0,6
0,4
0,2
0,0
Absorbance
0
200
400
600
800
Temps (s)
TiO2 non traité Ci = 0%
TiO2 traité Cl = 4,8%

FIG.7

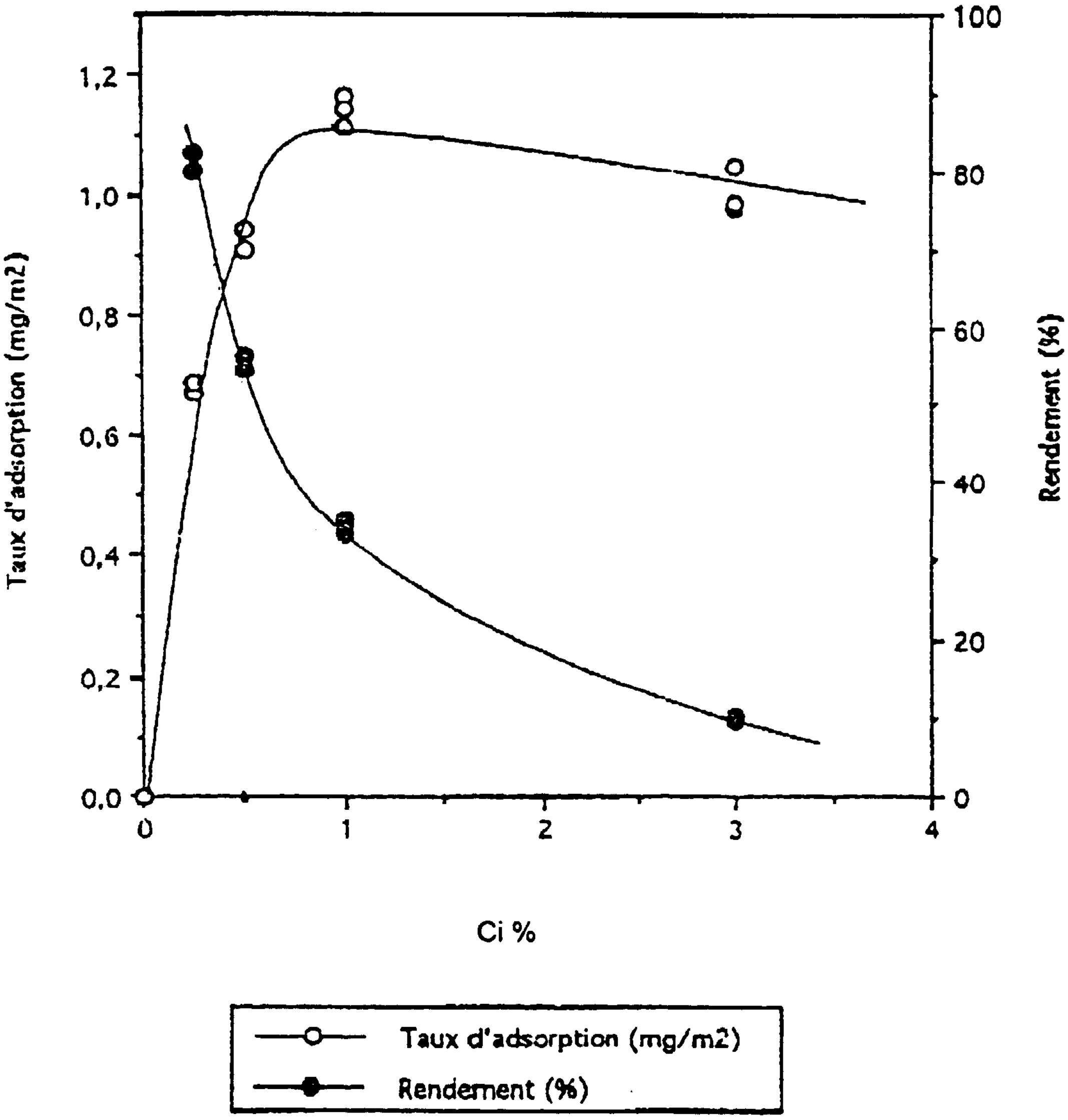
1,2
1,0
0,8
0,6
0,4
0,2
0,0
Taux d'adsorption (mg/m2)
100
80
60
40
20
0
Rendement (%)
0
1
2
3
4
Ci %
Taux d'adsorption (mg/m2)
Rendement (%)

**FIG.8**

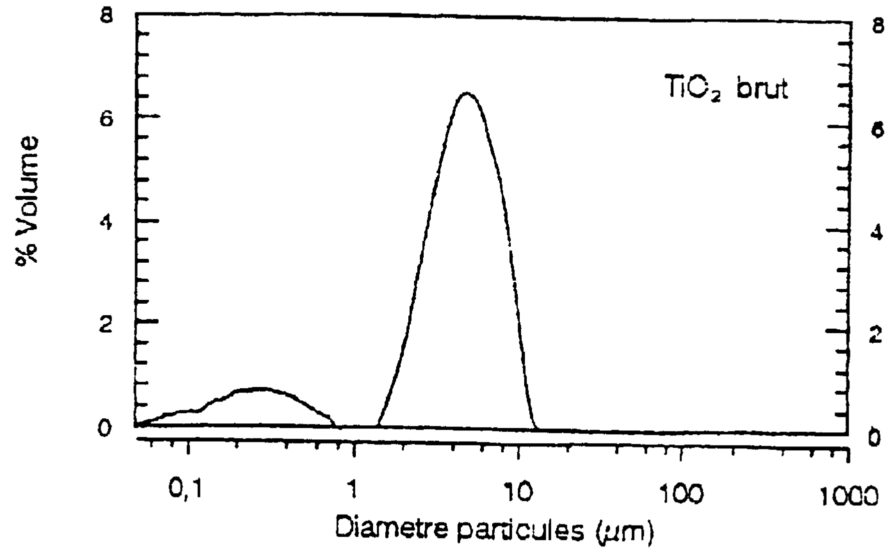


**FIG.9a**

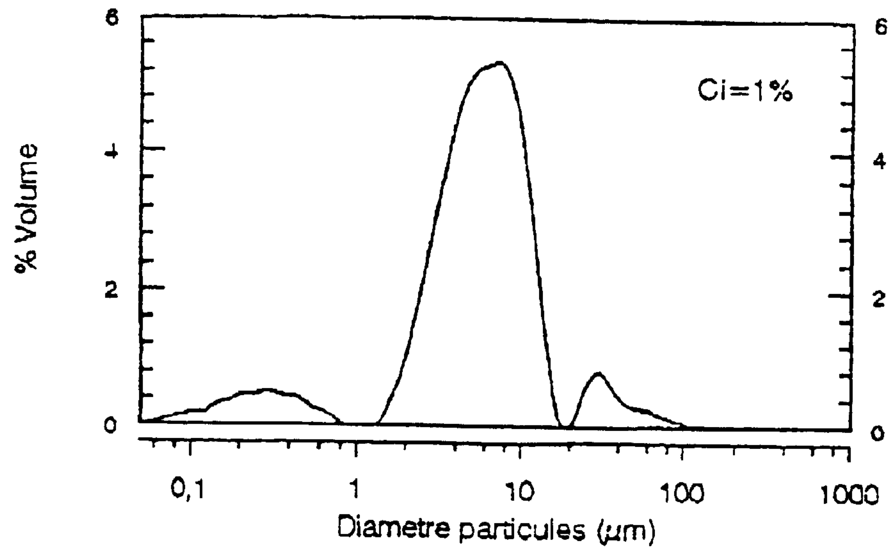


**FIG.9b**

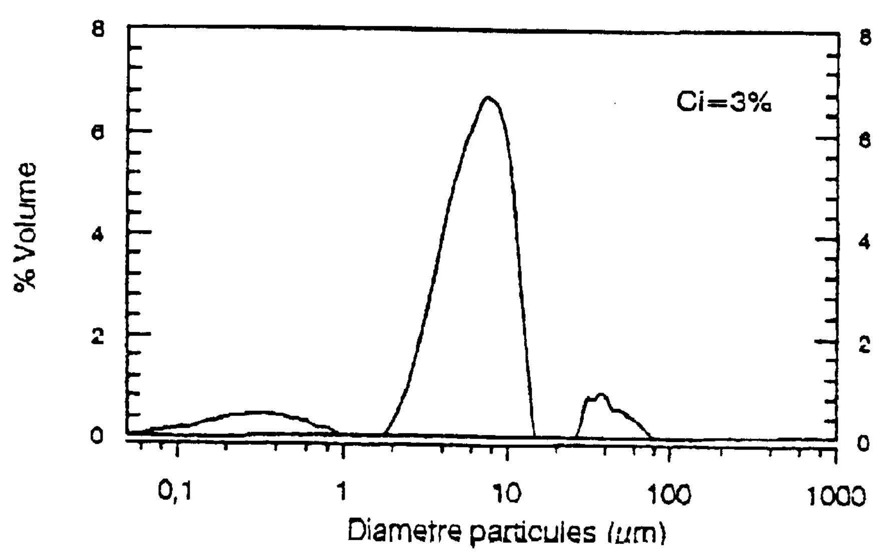


**FIG.9c**

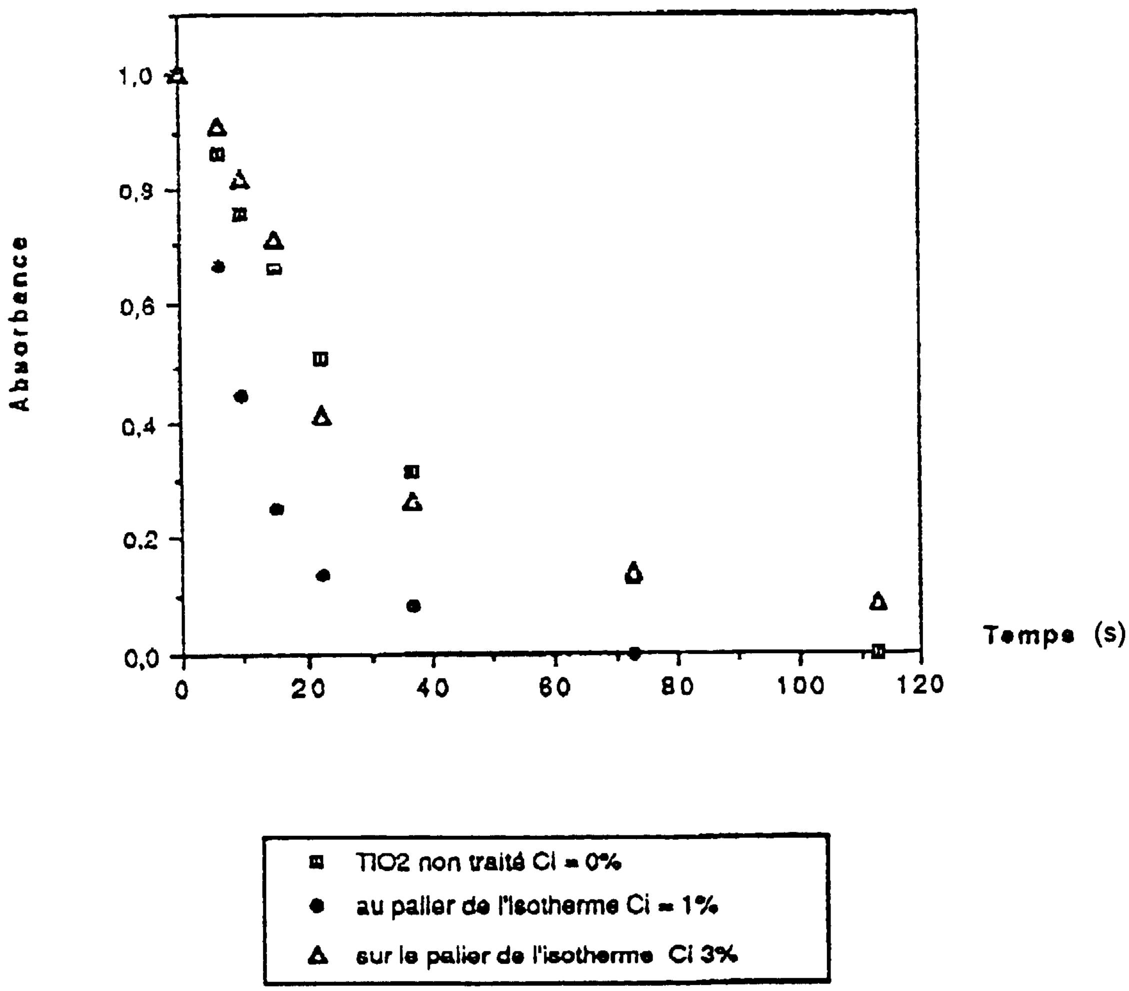
Absorbance
1,0
0,8
0,6
0,4
0,2
0,0
0
20
40
60
80
100
120
Temps (s)
TIO2 non traité Ci = 0%
au palier de l'isotherme Ci = 1%
sur le palier de l'isotherme Ci 3%

FIG.10